# EUROPEAN PATENT APPLICATION

(11) **EP 4 692 121 A1**
(43) Date of publication of application: **11.02.2026**
(21) Application number: 24780541.9
(22) Date of filing: 27.03.2024
(51) Int. Cl.: C07K 16/28, A61K 39/395, A61K 48/00, A61P 35/00, A61P 35/02, C12N 1/15, C12N 1/19, C12N 1/21, C12N 5/10, C12N 15/13

(54) **ANTIBODY, NUCLEIC ACID, CELL AND DRUG**

(30) Priority: 28.03.2023 JP 2023051486
(71) Applicant: NB Health Laboratory Co. Ltd., Sapporo-shi, Hokkaido 001-0021 (JP)
(72) Inventor: TAKASAKI Jun, Sapporo-shi, Hokkaido 001-0021 (JP); URUSHIBATA Yuji, Sapporo-shi, Hokkaido 001-0021 (JP); URUSHIBATA Kazuyo, Sapporo-shi, Hokkaido 001-0021 (JP); HAYASHI Yuki, Sapporo-shi, Hokkaido 001-0021 (JP); SASAKI Masako, Sapporo-shi, Hokkaido 001-0021 (JP)
(74) Representative: Ter Meer Steinmeister & Partner
(86) International application number: PCT/JP2024/012454
(87) International publication number: WO 2024/204440

(57) **Abstract**

The present invention provides an antibody that specifically binds to an extracellular domain of human CCR7 and has a heavy chain CDR1 including an amino acid sequence represented by SEQ ID NO: 25, a heavy chain CDR2 including an amino acid sequence represented by SEQ ID NO: 27, a heavy chain CDR3 including an amino acid sequence represented by SEQ ID NO: 29, a light chain CDR1 including an amino acid sequence represented by SEQ ID NO: 35, a light chain CDR2 including an amino acid sequence represented by SEQ ID NO: 37, and a light chain CDR3 including an amino acid sequence represented by SEQ ID NO: 39.

## Description

### TECHNICAL FIELD

This disclosure relates to an antibody that specifically binds to an extracellular domain of human CCR7, a nucleic acid encoding the antibody, a cell including the nucleic acid, and a medicament including the antibody as an active ingredient.

### BACKGROUND ART

A chemokine is a protein that modulates migration and cell functions of various cells. Functional abnormalities of a chemokine and its receptor cause various diseases such as fibrosis, autoimmune disease, acute and chronic inflammation, and cancer. Up to now, drugs that control the activities of a chemokine and its receptor have been developed and clinically applied, but it is hard to say that the problems are satisfactorily solved.

In order that a specific chemokine elicits activities such as modulation of cell migration and cell function, it is necessary that the chemokine binds to a chemokine-selective cell membrane receptor. About 20 kinds of chemokine receptors have been found, and all of these chemokine receptors are a seven transmembrane type protein (GPCR) that binds to a trimeric G protein. When a chemokine binds to a receptor, a Gα unit of a trimeric G protein is liberated. As a result, an intracellular Ca concentration increases, or phosphatidylinositol 3-kinase (PI3K) or small Rho GTPases pathways, or other pathways are activated, resulting in elicitation of the functions. Although all chemokine receptors are activated by a relatively selective chemokine, the receptors are very similar to each other in protein primary structures and intracellular activation mechanisms. Therefore, it is not easy to selectively block the function of a specific chemokine receptor. Elicitation of the functions by each chemokine and chemokine receptor in physiological and pathological conditions is controlled by each protein expressed during a specific period of time (during inflammation) in a specific cell or tissue (Non-Patent Document 1).

Human CC motif receptor 7 (CC MOTIF, RECEPTOR 7; also called EBI1, CMKBR7; hereinafter referred to as "CCR7") has been originally found as a GPCR that is expressed in a lymphocyte-selective manner by EPSTEIN-BARR virus infection (Non-Patent Document 2). Afterwards, CCR7 has been proved to be a selective chemokine receptor for CCL19 (also called ELC) and CCL21 (also called SLC, EXODUS 2). Under physiological conditions, CCR7 is expressed in cells such as CD4-positive T cells (Th1, Th2, Treg cells), mature dendritic cells, and B cells in a relatively selective manner. It is known that such cells are led to a lesion such as an inflamed site via CCR7 to enhance inflammatory reactions and immune responses. Abnormal activation of CCR7 causes various diseases such as autoimmune disease, fibrosis following acute and chronic inflammation, and cancer metastasis.

In cancer treatment, it is important to suppress proliferation of primary cancer and to prevent recurrence accompanying distant metastasis. In addition to conventional surgical therapies and chemical therapies, molecular target drugs (e.g. kinase inhibitor), antibody medicament therapies, and cancer immunotherapy have improved therapeutic outcomes, but development of a therapeutic drug for preventing recurrence accompanied by distant metastasis is increasingly desired. As a distant metastasis mechanism, a primary cancer spreads via blood vessels or lymphoid tissues. Although extracellular matrix protease inhibitors (MMP inhibitors), and the like have been developed as metastasis preventive drugs, there has been no case that the drugs are clinically applied.

Various studies have revealed that CCR7 is expressed in various tumor cells such as B cell chronic lymphocytic leukemia, non-Hodgkin's lymphoma, breast cancer cell, and malignant breast tumor. Further, it is becoming clear that CCR7 plays a role in lymph node metastasis of various cancers such as gastric cancer, melanoma, non-small-cell lung cancer, pulmonary adenocarcinoma, T cell leukemia cell, cervical cancer, various squamous cell cancer, hepatoma, urothelial cancer, and renal cell cancer (Non-Patent Document 1). Since CCL19 and CCL21 which are ligands for CCR7 are highly expressed in lymph nodes, it is expected that selective CCR7 function inhibition suppresses lymphatic metastasis of cancer cells.

As for a selective CCR7 function inhibitor, a low-molecular-weight compound, and a monoclonal antibody that selectively binds to an extracellular region of a receptor to block the CCR7 signaling mechanism induced by stimulation with CCL19/CCL21 are considered as candidates.

Patent Document 1 discloses eight monoclonal antibodies that specifically bind to an extracellular domain of human CCR7. Also, Patent Document 1 discloses amino acid sequences of a complementarity determining region (CDR), a heavy chain variable region, and a light chain variable region of each monoclonal antibody. These anti-human CCR7 antibodies are considered to be effective particularly for treatment of fibrosis.

Patent Document 2 discloses a humanized antibody of a type of monoclonal antibody that specifically binds to human CCR7. Also, Patent Document 2 discloses that the humanized antibody or an Fc variant with an enhanced complement-dependent cellular cytotoxicity kills multiple tumor cells of blood cancer by its effector function.

Patent Document 3 discloses an antibody-drug conjugate including an anticancer drug bound to a variant that is a monoclonal antibody specifically bondable with human CCR7 and has no effector function.

However, the antibodies described in Patent Documents 1 to 3 have not been practically used as therapeutic drugs. In the art, there is a demand for another or improved anti-human CCR7 antibody having superior characteristics as a pharmaceutical raw material.

The characteristics of the monoclonal antibody required as a raw material for an antibody pharmaceutical that inhibits the CCR7 function include high selectivity toward receptors, strong function-inhibiting activity, high solubility, thermal stability, low aggregability, low immunogenicity, and the like. All of these characteristics are required for the practical use of antibody medicaments, and in particular, the low immunogenicity for humans is important in terms of safety of antibody pharmaceuticals and determination of an administration period. The importance of the low immunogenicity is also explained in the guidelines on development of biopharmaceuticals, issued by United States Food and Drug Administration (FDA) and European Medicines Agency (EMA).

As a common method for decreasing immunogenicity of antibodies, the antibodies are humanized. Specifically, antibodies obtained from rodents can be humanized by a CDR grafting method while maintaining 100% of the amino acid sequences of the heavy chain CDRs 1 to 3 and the light chain CDRs 1 to 3, to decrease the immunogenicity while maintaining a high selectivity toward a receptor and a strong function-inhibiting activity. However, only two kinds of antibodies against GPCRs are marketed as pharmaceuticals, and it is not easy to decrease the immunogenicity while maintaining a high target molecule specificity and a strong function-inhibiting activity.

It is known that an antitumor activity of an antibody medicament in a living body is induced not only by the method for directly blocking an intracellular signaling mechanism of a receptor, but also by an antibody-dependent cell-mediated cytotoxicity (ADCC) activity or a complement-dependent cellular cytotoxicity (CDC) activity. The ADCC is a mechanism in which, when an antibody binds to an antigen on a cancer cell or target cell, immune cells such as macrophages and natural killer (NK) cells, which have an Fc receptor that recognizes an Fc region of the antibody, are attracted to the target cell to kill the cancer cell or target cell bound with the antibody. In cancer treatment, antibodies having a neutralizing activity as well as an ADCC activity are considered to be useful because they can exert an antitumor activity in a low dose. The intensity of the ADCC activity is determined depending on an amount of antigen expressed in a target cell, a strength of binding between an antigen and an antibody, selectivity of an antibody, and an intensity of affinity between an Fc region of an antibody and an Fc receptor. It is not clear that all GPCR antibodies have the ADCC activity, and the intensity of the ADCC activity varies for each antibody.

Examples of the methods for artificially enhancing the ADCC activity of the antibody include a method of modifying an amino acid sequence of the Fc region in the antibody and a method of controlling a sugar chain structure of the antibody. As the method of controlling a sugar chain structure of the antibody, a method of removing fucose at a reducing end of an N-type complex sugar chain of the antibody (defucosylation method) is known.

### PRIOR ART DOCUMENTS

### PATENT DOCUMENTS

Patent Document 1:WO 2012/043533
Patent Document 2: WO 2017/025569
Patent Document 3: WO 2021/220199

### NON-PATENT DOCUMENTS

Non-Patent Document 1: Viola A, Luster AD "Chemokines and their receptors: drug targets in immunity and inflammation", Annu Rev Pharmacol Toxicol. 2008;48:171-197
Non-Patent Document 2: Birkenbach, M., Josefsen, K., Yalamanchili, R., Lenoir, G., Kieff, E., "Epstein-Barr virus-induced genes: first lymphocyte-specific G protein-coupled peptide receptors", J. Virol. 67: 2209-2220, 1993.

### DISCLOSURE OF INVENTION

### TECHNICAL PROBLEM

As described above, the antibodies that inhibit the function of CCR7 are expected to be useful as antibody drugs, but have not been practically used as therapeutic drugs. Thus, an object of the present disclosure is to provide a novel anti-human CCR7 antibody having superior characteristics as a pharmaceutical raw material.

### SOLUTION TO PROBLEM

The present inventors performed humanization of the monoclonal antibody that blocks the CCR7 signaling mechanism described in Patent Document 1 by the CDR grafting method. However, this method did not make it possible to acquire the antibody having a desired low immunogenicity as a pharmaceutical. Thus, the present inventors investigated the function-inhibiting activity and the immunogenicity by variously modifying amino acid sequences of variable regions including CDRs constituting a known monoclonal antibody that blocks the CCR7 signaling mechanism. As a result, the present inventors succeeded in obtaining a novel anti-human CCR7 antibody including a CDR amino acid sequence different from those of existing antibodies and having low immunogenicity. Furthermore, it was found that the antibody had an effect of killing cancer cells, particularly leukemia cells with highly expressed CCR7 through the ADCC activity. Furthermore, the present inventors unexpectedly succeeded in enhancing the ADCC activity by defucosylating the sugar chain of the antibody.

The antibody according to an aspect of the present disclosure specifically binds to an extracellular domain of human CCR7, and has a heavy chain CDR1 including an amino acid sequence represented by SEQ ID NO: 25, a heavy chain CDR2 including an amino acid sequence represented by SEQ ID NO: 27, a heavy chain CDR3 including an amino acid sequence represented by SEQ ID NO: 29, a light chain CDR1 including an amino acid sequence represented by SEQ ID NO: 35, a light chain CDR2 including an amino acid sequence represented by SEQ ID NO: 37, and a light chain CDR3 including an amino acid sequence represented by SEQ ID NO: 39.

Preferably, the antibody has a heavy chain variable region including an amino acid sequence represented by SEQ ID NO: 21 and a light chain variable region including an amino acid sequence represented by SEQ ID NO: 31.

Preferably, the antibody has an Fc region, in which the Fc region includes an N-glycoside-bound sugar chain, fucose is not bound to position 6 of N-acetylglucosamine on a reducing end of the N-glycoside-bound sugar chain, and the antibody has an antibody-dependent cell-mediated cytotoxicity effect.

The antibody according to an aspect of the present disclosure specifically binds to an extracellular domain of human CCR7, and has: a heavy chain variable region including an amino acid sequence represented by SEQ ID NO: 21 with 1 to 10 amino acids substituted, added, or lacked, or including an amino acid sequence having at least 90% identity with the amino acid sequence represented by SEQ ID NO: 21; and a light chain variable region including an amino acid sequence represented by SEQ ID NO: 31 with 1 to 10 amino acids substituted, added, or lacked, or including an amino acid sequence having at least 90% identity with the amino acid sequence represented by SEQ ID NO: 31; as well as an Fc region, in which the Fc region includes an N-glycoside-bound sugar chain, fucose is not bound to position 6 of N-acetylglucosamine on a reducing end of the N-glycoside-bound sugar chain, and the antibody has an antibody-dependent cell-mediated cytotoxicity effect.

The antibody according to an aspect of the present disclosure specifically binds to the extracellular domain of human CCR7, and has a heavy chain CDR1 including an amino acid sequence represented by SEQ ID NO: 45, a heavy chain CDR2 including an amino acid sequence represented by SEQ ID NO: 47, a heavy chain CDR3 including an amino acid sequence represented by SEQ ID NO: 49, a light chain CDR1 including an amino acid sequence represented by SEQ ID NO: 55, a light chain CDR2 including an amino acid sequence represented by SEQ ID NO: 57, and a light chain CDR3 including an amino acid sequence represented by SEQ ID NO: 59.

Preferably, the antibody has a heavy chain variable region including an amino acid sequence represented by SEQ ID NO: 41, and a light chain variable region including an amino acid sequence represented by SEQ ID NO: 51.

Preferably, the antibody has an Fc region, in which the Fc region includes an N-glycoside-bound sugar chain, fucose is not bound to position 6 of N-acetylglucosamine on a reducing end of the N-glycoside-bound sugar chain, and the antibody has an antibody-dependent cell-mediated cytotoxicity effect.

The antibody according to an aspect of the present disclosure specifically binds to an extracellular domain of human CCR7, and has: a heavy chain variable region including an amino acid sequence represented by SEQ ID NO: 41 with 1 to 10 amino acids substituted, added, or lacked, or including an amino acid sequence having at least 90% identity with the amino acid sequence represented by SEQ ID NO: 41; and a light chain variable region including an amino acid sequence represented by SEQ ID NO: 51 with 1 to 10 amino acids substituted, added, or lacked, or including an amino acid sequence having at least 90% identity with the amino acid sequence represented by SEQ ID NO: 51; as well as an Fc region, in which the Fc region includes an N-glycoside-bound sugar chain, fucose is not bound to position 6 of N-acetylglucosamine on a reducing end of the N-glycoside-bound sugar chain, and the antibody has an antibody-dependent cell-mediated cytotoxicity effect.

A nucleic acid according to an aspect of the present disclosure encodes the above-described antibody.

Preferably, the nucleic acid includes a first nucleic acid encoding the amino acid sequence represented by SEQ ID NO: 21 and a second nucleic acid encoding the amino acid sequence represented by SEQ ID NO: 31.

Preferably, the nucleic acid includes a first nucleic acid encoding the amino acid sequence represented by SEQ ID NO: 41 and a second nucleic acid encoding the amino acid sequence represented by SEQ ID NO: 51.

A cell according to an aspect of the present disclosure includes the above-described nucleic acids.

A medicament according to an aspect of the present disclosure contains the above-described antibody as an active ingredient.

Preferably, the medicament is used for treating a cancer.

Preferably, the cancer is a hematological cancer.

Preferably, the hematological cancer is acute myeloid leukemia, acute lymphocytic leukemia, chronic myeloid leukemia, chronic lymphocytic leukemia, or non-Hodgkin's lymphoma.

Preferably, the non-Hodgkin's lymphoma is B cell lymphoblastic leukemia, T cell lymphoblastic leukemia, chronic lymphocytic leukemia, follicular lymphoma, MALT lymphoma, lymphoplasmacytic lymphoma, mantle cell lymphoma, diffuse large B cell lymphoma, Burkitt's lymphoma, mature T/NK cell-derived peripheral T cell lymphoma, adult T cell leukemia, extranodal NK/T cell lymphoma, or cutaneous lymphoma.

Preferably, the cancer is a solid carcinoma.

Preferably, the solid carcinoma is breast cancer, malignant breast tumor, gastric cancer, melanoma, non-small-cell lung cancer, pulmonary adenocarcinoma, cervical cancer, hepatoma, urothelial cancer, renal cell cancer, or squamous cell cancer.

Preferably, the squamous cell cancer is oral squamous cell carcinoma, esophageal squamous cell carcinoma, or pharyngeal squamous cell carcinoma.

### EFFECT OF INVENTION

The present disclosure makes it possible to provide a novel anti-human CCR7 antibody having superior characteristics as a pharmaceutical raw material.

### BRIEF DESCRIPTION OF DRAWINGS

Fig. 1A is an explanatory diagram presenting alignments of heavy chain variable regions of a plurality of humanized anti-CCR7 antibodies obtained in Examples.
Fig. 1B is an explanatory diagram presenting alignments of light chain variable regions of a plurality of humanized anti-CCR7 antibodies obtained in Examples.
Fig. 2 is a graph presenting a relationship between an inhibitory activity of each antibody on an intracellular Ca²⁺ signaling and an antibody concentration.
Fig. 3 is a graph presenting a number of donors positive for immunogenicity of each antibody.
Fig. 4A is a graph presenting a relationship between an antibody concentration and a fluorescence intensity of a flow cytometry histogram in an hCCR7 gene-transfected cell.
Fig. 4B is a graph presenting a relationship between an antibody concentration and a fluorescence intensity of a flow cytometry histogram in Granta-519 cell.
Fig. 4C is a graph presenting a relationship between an antibody concentration and a fluorescence intensity of a flow cytometry histogram in MJ cell.
Fig. 5A is a graph presenting a result of evaluating an inhibitory activity of NB007-01 on an intracellular Ca²⁺ signaling in a case that an hCCR7 gene-transfected cell is stimulated with CCL21.
Fig. 5B is a graph presenting a result of evaluating an inhibitory activity of NB007-01 on an intracellular Ca²⁺ signaling in a case that an hCCR7 gene-transfected cell is stimulated with CCL19.
Fig. 6A is a graph presenting a result of evaluating an inhibitory activity of NB007-01 on an intracellular Ca²⁺ signaling in a case that an MJ cell is stimulated with CCL21.
Fig. 6B is a graph presenting a result of evaluating an inhibitory activity of CAP-100 on an intracellular Ca²⁺ signaling in a case that an MJ cell is stimulated with CCL21.
Fig. 7 is a graph presenting a result of evaluating an inhibitory activity of NB0007-01 on a CCL19-induced cell migration in a Granta-519 cell.
Fig. 8A is an HPLC chromatogram presenting a result of analyzing a sugar chain structure of a defucosylated NB007-01.
Fig. 8B is an explanatory diagram presenting a percentage of each sugar chain type, calculated from the chromatogram in Fig. 8A.
Fig. 9 is a graph presenting a result of evaluating a cytotoxicity function of each antibody by an ADCC Reporter Bioassay method.
Fig. 10 is a graph presenting a result of evaluating a cytotoxicity function of each antibody by a cytotoxicity activity measurement method using human PBMC.
Fig. 11 is a graph presenting a result of evaluating an antitumor effect of each antibody in a Granta-519 cell-transplanted model.
Fig. 12A is a graph presenting a result of evaluating an effect of each antibody for suppressing cell infiltration into a lymph node in a Granta-519 cell-transplanted model.
Fig. 12B is a graph presenting a result of evaluating an effect of each antibody for suppressing cell infiltration into a liver in a Granta-519 cell-transplanted model.

### BEST MODE FOR CARRYING OUT THE INVENTION

In the present disclosure, the complementarity determining region is abbreviated as CDR. In the present disclosure, the heavy chain variable region is abbreviated as VH, the heavy chain constant region as CH, the light chain variable region as VL, and the light chain constant region as CL, in some cases. In the present disclosure, the term "antibody" may be replaced with "immunoglobulin". In the present disclosure, the term "nucleic acid" may be replaced with "DNA" or "gene".

In the present disclosure, the humanized antibody refers to an antibody with a CDR derived from an animal other than humans, and with the other regions (e.g. framework regions and constant regions) derived from humans. In the present disclosure, the chimeric antibody refers to an antibody with a heavy chain variable region (VH) and light chain variable region (VL) derived from an animal other than humans, and with the other regions such as a heavy chain constant region (CH) and light chain constant region (CL) derived from humans.

### <Human CCR7>

CCR7 is a type of G-protein coupled receptor (GPCR) that penetrates a cell membrane seven times and has an N-terminal directed to the outside of the cell and a C-terminal directed to the inside of the cell. The gene (cDNA) encoding human CCR7 has already been isolated, and the amino acid sequence of human CCR7 is also known. Information of the amino acid sequence can be obtained, for example, from database of GenBank or the like (e.g. GenBank: EAW60669.1). As an example, a nucleotide sequence of human CCR7 gene is presented in SEQ ID NO: 81. An amino acid sequence encoded by the nucleotide sequence is presented in SEQ ID NO: 82.

Each domain in human CCR7 is considered to correspond to each of the following parts in the amino acid sequence represented by SEQ ID NO: 82. The amino acid numbers are described on the left, and the domains are described on the right. Note that borders between the domains are slightly movable back and forth.
1 to 24: Membrane translocation signal peptide sequence (cut and removed after expression)
25 to 59: N-terminal domain
87 to 95: Intracellular first loop domain
117 to 130: Extracellular first loop domain
153 to 170: Intracellular second loop domain
192 to 219: Extracellular second loop domain
248 to 263: Intracellular third loop domain
290 to 313: Extracellular third loop domain
332 to 378: C-terminal domain

As the human CCR7, besides the amino acid sequence represented by SEQ ID NO: 82, various variants such as amino acid substitutes are known. The "human CCR7" in the present disclosure includes the variants as long as it has an extracellular domain and functionally serves as CCR7.

### <Anti-CCR7 Antibody>

The antibody disclosed in this application specifically binds to the extracellular domain of human CCR7. The antibody according to an aspect has a heavy chain CDR1 including an amino acid sequence represented by SEQ ID NO: 25, a heavy chain CDR2 including an amino acid sequence represented by SEQ ID NO: 27, a heavy chain CDR3 including an amino acid sequence represented by SEQ ID NO: 29, a light chain CDR1 including an amino acid sequence represented by SEQ ID NO: 35, a light chain CDR2 including an amino acid sequence represented by SEQ ID NO: 37, and a light chain CDR3 including an amino acid sequence represented by SEQ ID NO: 39. Preferably, the antibody is a humanized antibody having a heavy chain variable region including an amino acid sequence represented by SEQ ID NO: 21 and a light chain variable region including an amino acid sequence represented by SEQ ID NO: 31. The antibody according to this aspect is exemplified by NB007-01 described in Examples below.

The heavy chain variable region (SEQ ID NO: 21) includes the heavy chain CDRs 1 to 3 (SEQ ID NOs: 25, 27, and 29), and regions other than the CDRs are derived from human antibodies. Similarly, the light chain variable region (SEQ ID NO: 31) includes the light chain CDRs 1 to 3 (SEQ ID NOs: 35, 37, and 39), and regions other than the CDRs are derived from human antibodies.

An antibody according to another aspect has a heavy chain CDR1 including an amino acid sequence represented by SEQ ID NO: 45, a heavy chain CDR2 including an amino acid sequence represented by SEQ ID NO: 47, a heavy chain CDR3 including an amino acid sequence represented by SEQ ID NO: 49, a light chain CDR1 including an amino acid sequence represented by SEQ ID NO: 55, a light chain CDR2 including an amino acid sequence represented by SEQ ID NO: 57, and a light chain CDR3 including an amino acid sequence represented by SEQ ID NO: 59. Preferably, the antibody is a humanized antibody having a heavy chain variable region including an amino acid sequence represented by SEQ ID NO: 41 and a light chain variable region including an amino acid sequence represented by SEQ ID NO: 51. The antibody according to this aspect is exemplified by NB007-02 described in Examples below.

The heavy chain variable region (SEQ ID NO: 41) includes the heavy chain CDRs 1 to 3 (SEQ ID NOs: 45, 47, and 49), and regions other than the CDRs are derived from human antibodies. Similarly, the light chain variable region (SEQ ID NO: 51) includes the light chain CDRs 1 to 3 (SEQ ID NOs: 55, 57, and 59), and regions other than the CDRs are derived from human antibodies.

Furthermore, the present disclosure encompasses an antibody that specifically binds to the extracellular domain of human CCR7, which has a heavy chain CDR1 including an amino acid sequence represented by SEQ ID NO: 5, a heavy chain CDR2 including an amino acid sequence represented by SEQ ID NO: 7, a heavy chain CDR3 including an amino acid sequence represented by SEQ ID NO: 9, a light chain CDR1 including an amino acid sequence represented by SEQ ID NO: 15, a light chain CDR2 including an amino acid sequence represented by SEQ ID NO: 17, and a light chain CDR3 including an amino acid sequence represented by SEQ ID NO: 19. Preferably, the antibody is a chimeric antibody having a heavy chain variable region including an amino acid sequence represented by SEQ ID NO: 1 and a light chain variable region including an amino acid sequence represented by SEQ ID NO: 11. The antibody is exemplified by VH0/VL0 described in Examples below.

Furthermore, the present disclosure encompasses an antibody that specifically binds to the extracellular domain of human CCR7, which has a heavy chain CDR1 including an amino acid sequence represented by SEQ ID NO: 65, a heavy chain CDR2 including an amino acid sequence represented by SEQ ID NO: 67, a heavy chain CDR3 including an amino acid sequence represented by SEQ ID NO: 69, a light chain CDR1 including an amino acid sequence represented by SEQ ID NO: 75, a light chain CDR2 including an amino acid sequence represented by SEQ ID NO: 77, and a light chain CDR3 including an amino acid sequence represented by SEQ ID NO: 79. Preferably, the antibody is a humanized antibody having a heavy chain variable region including an amino acid sequence represented by SEQ ID NO: 61 and a light chain variable region including an amino acid sequence represented by SEQ ID NO: 71. The antibody is exemplified by NB007-03 described in Examples below.

The antibody may be a functional fragment. Here, the "functional fragment of antibody" refers to a partial fragment of the antibody (i.e. immunoglobulin) having at least one effect on an antigen. Examples of the partial fragment include F(ab')2, Fab, Fv, disulfide-bonded Fv, single chain antibody (scFv, VH-VL). Furthermore, the antibody according to the present disclosure may be a multispecific antibody such as a diabody.

When the antibody is a functional fragment, for example, the effect as will be described later is achieved. That is, when a full-length antibody such as type IgG is used for applying the anti-human CCR7 antibody according to the present disclosure to a medicament as will be described later, the signaling of the target receptor may be inhibited, and furthermore the target tissue may be damaged, leading to a side effect. In such a case, by adopting the "functional fragment of an antibody" using only a variable region, the side effect as described above is easily avoided.

Preferably, the antibody has an activity of blocking a CCR7-dependent intracellular signaling mechanism caused by stimulation with CCR7 ligand.

Preferably, the antibody has an antibody-dependent cell-mediated cytotoxicity (ADCC) activity. This makes the antibody particularly suitable as an active ingredient in a cancer therapeutic drug.

The present disclosure encompasses an antibody that is "functionally equivalent" to the above-described anti-human CCR7 antibody. For example, an antibody that specifically binds to the extracellular domain of human CCR7 is disclosed, which has: a heavy chain variable region including an amino acid sequence represented by SEQ ID NO: 21 with 1 to 10 amino acids substituted, added, or lacked, or including an amino acid sequence having at least 90% identity with the amino acid sequence represented by SEQ ID NO: 21; and a light chain variable region including an amino acid sequence represented by SEQ ID NO: 31 with 1 to 10 amino acids substituted, added, or lacked, or including an amino acid sequence having at least 90% identity with the amino acid sequence represented by SEQ ID NO: 31, and has an antibody-dependent cell-mediated cytotoxicity activity.

Similarly, an antibody that specifically binds to the extracellular domain of human CCR7 is disclosed, which has: a heavy chain variable region including an amino acid sequence represented by SEQ ID NO: 41 with 1 to 10 amino acids substituted, added, or lacked, or including an amino acid sequence having at least 90% identity with the amino acid sequence represented by SEQ ID NO: 41; and a light chain variable region including an amino acid sequence represented by SEQ ID NO: 51 with 1 to 10 amino acids substituted, added, or lacked, or including an amino acid sequence having at least 90% identity with the amino acid sequence represented by SEQ ID NO: 51, and has an antibody-dependent cell-mediated cytotoxicity activity.

The number of substituted, added, or lacked amino acids described above is preferably 1 to 8, more preferably 1 to 5, particularly preferably 1 to 3. The amino acid sequence identity described above is preferably at least 92%, more preferably at least 95%, particularly preferably at least 97%.

### <Defucosylation of Fc Region>

It is preferable that the anti-human CCR7 antibody has an Fc region, the Fc region includes an N-glycoside-bound sugar chain, and fucose is not bound to position 6 of N-acetylglucosamine on a reducing end of the N-glycoside-bound sugar chain. Simply stated, it is preferable that the N-glycoside-bound sugar chain in the Fc region is defucosylated. Furthermore, in other words, it is preferable that a core fucose is not bound to the N-glycoside-bound sugar chain in the Fc region. It is expected that the ADCC activity of the antibody is enhanced and the immunogenicity of the antibody is lowered by defucosylating the N-glycoside-bound sugar chain in the Fc region.

In a method for preparing the antibody with the defucosylated N-glycoside-bound sugar chain in the Fc region (defucosylated antibody), for example, a host cell with a lowered or lacked enzyme activity involved in fucose synthesis is used. Examples of the enzyme include GDP-mannose 4,6-dehydratase (GMD), GDP-keto-6-deoxymannose 3,5-epimerase, 4-reductase (Fx), GDP-β-L-fucose pyrophosphorylase (GFPP), and α-1,6-fucosyltransferase (FUT8). Examples of the host cell include CHO cells.

When the defucosylated antibody is actually produced, the defucosylated antibody is obtained as an antibody composition including a mixture of defucosylated antibodies and non-defucosylated antibodies in some cases. In the antibody composition, a ratio (molar ratio) of the defucosylated antibodies and the non-defucosylated antibodies is not particularly limited, but the defucosylated antibodies account for preferably 50% or higher, more preferably 70% or higher, particularly preferably 80% or higher. In other words, in the antibody composition, preferably 50% or higher, more preferably 70% or higher, particularly preferably 80% or higher of N-glycoside-bound sugar chains are defucosylated.

### <Nucleic Acid>

The present disclosure encompasses a nucleic acid (DNA) encoding the above-described antibody. The nucleic acid includes, for example, a first nucleic acid encoding the heavy chain variable region and/or a second nucleic acid encoding the light chain variable region. Specific examples of the nucleic acids include a nucleic acid including a first nucleic acid encoding the amino acid sequence represented by SEQ ID NO: 21 and a second nucleic acid encoding the amino acid sequence represented by SEQ ID NO: 31, as well as a nucleic acid including a first nucleic acid encoding the amino acid sequence represented by SEQ ID NO: 41 and a second nucleic acid encoding the amino acid sequence represented by SEQ ID NO: 51.

The above nucleic acids may be incorporated into a vector. The vector is selected as appropriate depending on a type of a host cell into which the vector is introduced, or the like. The vector includes gene therapy vectors.

### <Cell>

The present disclosure encompasses a cell including the above-described nucleic acids. The cell is exemplified by a cell including a vector into which the nucleic acids have been incorporated. The type of the cell is not particularly limited as long as the cell can express the above-described nucleic acids, and, for example, the above-described vector functions in the cell. Examples of the cell include animal cells (e.g. COS cell, CHO cell), yeast, bacteria (e.g. Escherichia coli), plant cells, and insect cells.

### <Method for Producing Antibody>

The above-described antibody can be produced using a genetic recombination technique. That means, a recombinant cell that expresses the above-described nucleic acid can be constructed to obtain the antibody from a culture of the cell.

A method for constructing and producing a humanized antibody will be explained as an example. First, as the DNAs encoding the heavy chains CDR1 to 3 and the light chains CDR1 to 3, each DNA encoding amino acid sequences represented by SEQ ID NO: 25, 27, 29, 35, 37, or 39 is prepared. The DNAs are exemplified by nucleotide sequences represented by SEQ ID NO: 26, 28, 30, 36, 38, and 40, but may also be other nucleotide sequences.

In another example, as the DNAs encoding the heavy chains CDR1 to 3 and the light chains CDR1 to 3, each DNA encoding amino acid sequences represented by SEQ ID NO: 45, 47, 49, 55, 57, or 59 is prepared. The DNAs are exemplified by nucleotide sequences represented by SEQ ID NO: 46, 48, 50, 56, 58, and 60, but may also be other nucleotide sequences.

Next, using these DNAs, a DNA is prepared, which encodes a variable region where the heavy chains CDR1 to 3 are transplanted into a framework region (FR) in a VH of any human antibody. Similarly, a DNA is prepared, which encodes a variable region where the light chains CDR1 to 3 are transplanted into an FR in a VL of any human antibody. Each of the prepared DNAs is inserted into a vector having a sequence encoding a CH or CL of a human antibody to construct a humanized antibody expression vector. The constructed expression vector is introduced into a host cell to obtain a recombinant cell that expresses a humanized antibody. Then, the recombinant cell is cultured, and a desired humanized antibody is obtained from the culture.

The method for purifying the antibody is not particularly limited, and any known method may be adopted. For example, the culture supernatant of the recombinant cell is collected, from which the antibody can be purified by a combination of known methods such as various chromatographies, salt precipitation, dialysis, and membrane separation.

### <Medicament>

The present disclosure encompasses a medicament containing the above-described anti-human CCR7 antibody as an active ingredient. The medicament may be a pharmaceutical composition containing the above-described anti-human CCR7 antibody and a pharmaceutically acceptable carrier. Preferably, the medicament blocks the CCR7-dependent intracellular signaling mechanism induced by stimulation with the CCR7 ligand. Preferably, the medicament has an antibody-dependent cell-mediated cytotoxicity (ADCC) activity.

In a preferable aspect, the above-described medicament is used for cancer treatment. For example, the medicament is used as an anticancer drug. The cancer may be either a hematological cancer or a solid carcinoma.

Examples of hematological cancer include acute myeloid leukemia, acute lymphocytic leukemia, chronic myeloid leukemia, chronic lymphocytic leukemia, and non-Hodgkin's lymphoma. Examples of non-Hodgkin's lymphoma include B cell lymphoblastic leukemia, T cell lymphoblastic leukemia, chronic lymphocytic leukemia, follicular lymphoma, MALT lymphoma, lymphoplasmacytic lymphoma, mantle cell lymphoma, diffuse large B cell lymphoma, Burkitt's lymphoma, mature T/NK cell-derived peripheral T cell lymphoma, adult T cell leukemia, extranodal NK/T cell lymphoma, and cutaneous lymphoma

Examples of solid carcinoma include breast cancer, malignant breast tumor, gastric cancer, melanoma, non-small-cell lung cancer, pulmonary adenocarcinoma, cervical cancer, hepatoma, urothelial cancer, renal cell cancer, and squamous cell cancer. Examples of squamous cell cancer include oral squamous cell carcinoma, esophageal squamous cell carcinoma, and pharyngeal squamous cell carcinoma.

Herein, "treatment" means prevention or alleviate of progression and exacerbation of a disease symptom in a mammal that is at risk of suffering from or has suffered from the disease. Thereby, the "treatment" is used to mean a therapeutic treatment aimed to prevent or alleviate progression and exacerbation of various symptoms and the like of the disease.

### <Administration Method>

The medicament can be administered systemically or topically, in an oral route or a parenteral route. Examples of a dosage form include an injection form, a nasal dosage form, a pulmonary dosage form, and a transdermal dosage form. In the case of an injection form, the medicament may be systemically or topically administered, for example, by intravenous injection, intramuscular injection, intraperitoneal injection, or subcutaneous injection. The administration method may be selected as appropriate depending on an age and a symptom of a patient. A dose of the antibody may be selected e.g. within a range of 0.0001 mg to 1000 mg per 1 kg of body weight per one dosage. Alternatively, the dose can be selected e.g. within a range of 0.001 to 100,000 mg per one patient. However, the dose of the antibody is not limited to these ranges.

### <Formulation>

The above medicament can be formulated according to an ordinary method (e.g. Remington's Pharmaceutical Science, latest edition, Mark Publishing Company, Easton, U.S.A.). The medicament can contain pharmaceutically acceptable carriers or additives. Examples of the carriers or additives include, but are not limited to, surfactants (e.g. PEG, Tween), excipients, antioxidants (e.g. ascorbic acid), colorants, fragrant agents, preservatives, stabilizers, buffers (e.g. phosphoric acid, citric acid, other organic acids), chelating agents (e.g. EDTA), suspensions, isotonizing agents, binders, disintegrators, lubricants, fluidity accelerators, and flavoring agents, and other commonly used carriers or the like can be used as appropriate. Specific examples of the carriers or additives include light anhydrous silicic acid, lactose, crystalline cellulose, mannitol, starch, carmellose calcium, carmellose sodium, hydroxypropyl cellulose, hydroxypropylmethyl cellulose, polyvinylacetal diethylaminoacetate, polyvinylpyrrolidone, gelatin, medium-chain fatty acid triglyceride, polyoxyethylene hardened castor oil 60, saccharose, carboxymethyl cellulose, corn starch, and inorganic salts. The medicament may contain other low-molecular-weight polypeptides; proteins such as serum albumin, gelatin, and immunoglobulin; and amino acids such as glycine, glutamine, asparagine, arginine, and lysine.

When the medicament is used in a form of an aqueous solution for injection, the aqueous solution may be saline, or an isotonic solution containing glucose or other auxiliary agents, e.g. a solution of D-sorbitol, D-mannose, D-mannitol, or sodium chloride, and may be used in combination with an appropriate solubilizer, e.g. an alcohol (e.g. ethanol), a polyalcohol (e.g. propylene glycol, PEG), a nonionic surfactant (polysorbate 80, HCO-50), or the like. If necessary, the above antibodies can be encapsulated in microcapsules (e.g. made of hydroxymethyl cellulose, gelatin, poly[methyl methacrylate]), or can be configured as a colloidal drug delivery system (e.g. liposomes, albumin microspheres, microemulsions, nanoparticles, and nanocapsules) (e.g. see "Remington's Pharmaceutical Science 16th edition", Oslo Ed., 1980).

Furthermore, techniques for sustained release of drugs are known and can be applied to the above medicament (Langer et al., J. Biomed. Master. Res. 15: 162-277 (1981); Langer, Chem. Tech. 12:98-105 (1982); US Patent No. 3,773,919; EP Patent Application Publication No. 58,481; Sidman et al., Biopolymers 22: 547-556 (1983); EP Patent Application Publication No. 133,988).

The above-described medicament can be administered as an antibody-drug conjugate (ADC). Examples of the drug in combination with the antibody include antitumor drugs monomethylauristatin E (MMAE) and monomethylauristatin F (MMAF).

### <Application to Gene Therapy>

It is also conceivable to incorporate the above-described nucleic acids into a vector for a gene therapy to prepare a gene therapeutic drug. Examples of a method for administering the gene therapeutic drug (recombinant vector) include a direct administration using a naked plasmid, as well as a method of packaging the drug in liposome or the like, a method of incorporating the drug into various viral vectors such as retroviral vectors, adenoviral vectors, vaccinia virus vectors, poxvirus vectors, adeno-associated virus vectors, and HVJ vectors (see, Adolph "Viral Genome Methods", CRC Press, Florid (1996)), and a method of coating a bead carrier such as a colloidal gold particle with the drug (WO 93/17706 etc.).

The gene therapeutic drug may be administered in any method as long as the antibody is expressed in a living body and is able to exert its effect. Preferably, a sufficient amount of the drug is administered through an appropriate parenteral route, such as intravenous route, intraperitoneal route, subcutaneous route, intradermal route, intra-adipose route, intramammary route, inhalation, intramuscular route, or the like by a method using injection, infusion, gas inductive particle bombardment (e.g. using an electron gun), mucosal route using collunarium or the like. Furthermore, the gene therapeutic drug may be administered ex vivo by a process in which the drug is administered into cells using liposome transfection, particle bombardment (U.S. Patent No. 4,945,050), or viral infection, and the cells are reintroduced into an animal.

### <Other Disclosures>

The present disclosure encompasses a method for treating a cancer, including administering an effective amount of the above antibodies to a cancer patient. The present disclosure encompasses the above antibodies for use in treating a cancer. The present disclosure encompasses use of the above antibodies for producing a medicament for use in treating a cancer.

### EXAMPLES

### [Example 1] Preparation of Humanized Anti-CCR7 Antibody

In order to prepare a humanized antibody of a mouse anti-CCR7 antibody R7-18 (described in WO 2012/043533 and JP Patent No. 5315495), a chimeric anti-CCR7 antibody "VH0/VL0" was first prepared by fusing heavy chain/light chain variable regions of R7-18 with an Fc region of a human antibody IgG1. Furthermore, a human framework was selected based on a homology between R7-18 and human germline VH/VK genes. Based on a computer modeling, a plurality of variable region sequences were designed with variation in sequences of the selected human germline VH and VK or the sequence of the CDR of the transplanted R7-18, so that a predicted antibody conformation of R7-18 could be supported. Among the designed humanized antibodies, "VH5-2/VL1-3", "VH5-1/VL1", and "VH5/VL5", which had maintained CCR7 neutralizing activity were selected, and named "NB007-01", "NB007-02", and "NB007-03" respectively. NB007-01, NB007-02, NB007-03, and a reference antibody CAP-100 (described in Patent Document 2) were prepared using a QMCF method (described in WO 2006/084754 and JP 2008-529510 A).

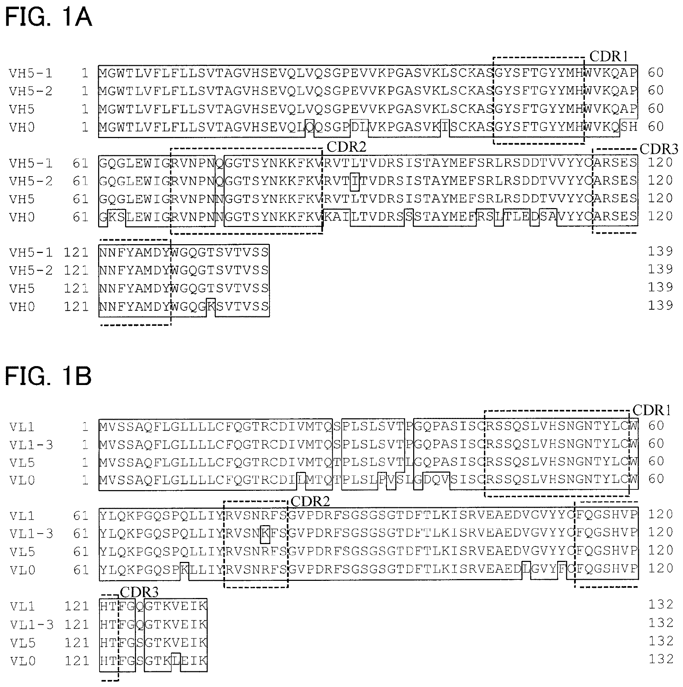

Alignments of the heavy chain variable region VH0 (SEQ ID NO: 1) of VH0/VL0, the designed heavy chain variable region VH5-2 (SEQ ID NO: 21) of NB007-01, the designed heavy chain variable region VH5-1 (SEQ ID NO: 41) of NB007-02, and the designed heavy chain variable region VH5 (SEQ ID NO: 61) of NB007-03 are presented in Fig. 1A. Alignments of the light chain variable region VL0 (SEQ ID NO: 11) of VH0/VL0, the designed light chain variable region VL1-3 (SEQ ID NO: 31) of NB007-01, the designed light chain variable region VL1 (SEQ ID NO: 51) of NB007-02, and the designed light chain variable region VL5 (SEQ ID NO: 71) of NB007-03 are presented in Fig. 1B. In Fig. 1A and Fig. 1B, the regions that cover all CDRs defined by KABAT method and IMGT method are surrounded by dashed line boxes.

The amino acid sequences (AA) of the heavy chain variable region, the heavy chain constant region, the heavy chain CDRs 1 to 3, the light chain variable region, the light chain constant region, and the light chain CDRs 1 to 3 of each antibody obtained, as well as the nucleotide sequences of the DNAs encoding them, are summarized in Tables 1-1 to 1-8.

**[Table 1-1]**

| TABLE 1-1 VH0/VL0 (heavy chain) | | | |
|---|---|---|---|
| SEQ ID NO: | Region | Type | Sequence |
| 1 | Heavy chain variable region | AA | MGWTLVFLFLLSVTAGVHSEVQLQQSGPDLVKPGASVKISCKASGYSFTGYY MHWVKQSHGKSLEWIGRVNPNNGGTSYNKKFKVKAILTVDRSSSTAYMEFRS LTLEDSAVYYCARSESNNFYAMDYWGQGKSVTVSS |
| 2 | Heavy chain variable region | DNA | atgggctggactttggtgttcctctttctgctgtctgtcactgctggtgtac actccgaagtgcagcttcagcagtcaggacctgacttggtgaaaccaggtgc tagtgtcaagatcagctgcaaagcaagcgggtatagctttaccggctattac atgcactgggtgaaacagagccatggaagtccctggaatggattgggaggg ttaatcccaataatggaggcacatcctacaacaagaagttcaaggtgaaagc catcctgacggttgatcggtccagttctaccgcctacatggagtttcgcagt ctcacactggaggattctgctgtctactactgtgcaagatcagagtccaaca acttctatgccatggactattggggacaaggcaagagcgtaaccgtgtcatc t |
| 3 | Heavy chain constant region | AA | |
| 4 | Heavy chain constant region | DNA | gctagcaccaagggcccatcggtcttccccctggcaccctcctccaagagca cctctgggggcacagcggccctgggctgcctggtcaaggactacttccccga accggtgacggtgtcgtggaactcaggcgccctgaccagcggcgtgcacacc ttcccggctgtcctacagtcctcaggactctactccctcagcagcgtggtga ccgtgccctccagcagcttgggcacccagacctacatctgcaacgtgaatca caagcccagcaacaccaaggtggacaagaaagttgagcccaaatcttgtgac aaaactcacacatgcccaccgtgcccagcacctgaactcctggggggaccgt cagtcttcctcttccccccaaaacccaaggacaccctcatgatctcccggac ccctgaggtcacatgcgtggtggtggacgtgagccacgaagaccctgaggtc aagttcaactggtacgtggacggcgtggaggtgcataatgccaagacaaagc cgcgggaggagcagtacaacagcacgtaccgtgtggtcagcgtcctcaccgt cctgcaccaggactggctgaatggcaaggagtacaagtgcaaggtctccaac aaagccctcccagcccccatcgagaaaaccatctccaaagccaaagggcagc cccgagaaccacaggtgtacaccctgcccccatcccgggatgagctgaccaa gaaccaggtcagcctgacctgcctggtcaaaggcttctatcccagcgacatc gccgtggagtgggagagcaatgggcagccggagaacaactacaagaccacgc ctcccgtgctggactccgacggctccttcttcctctacagcaagctcaccgt ggacaagagcaggtggcagcaggggaacgtcttctcatgctccgtgatgcat gaggctctgcacaaccactacacgcagaagagcctctccctgtctccgggta aatga |
| 5 | Heavy chain CDR1 | AA | GYSFTGYYMH |
| 6 | Heavy chain CDR1 | DNA | gggtatagctttaccggctattacatgcac |
| 7 | Heavy chain CDR2 | AA | RVNPNNGGTSYNKKFKV |
| 8 | Heavy chain CDR2 | DNA | agggttaatcccaataatggaggcacatcctacaacaagaagttcaaggtg |
| 9 | Heavy chain CDR3 | AA | ARSESNNFYAMDY |
| 10 | Heavy chain CDR3 | DNA | gcaagatcagagtccaacaacttctatgccatggactat |

**[Table 1-2]**

| TABLE 1-2 VH0/VL0(light chain) | | | |
|---|---|---|---|
| SEQ ID NO: | Region | Type | Sequence |
| 11 | Light chain variable region | AA | MVSSAQFLGLLLLCFQGTRCDILMTQTPLSLPVSLGDQVSISCRSSQSLVHS NGNTYLCWYLQKPGQSPKLLIYRVSNRFSGVPDRFSGSGSGTDFTLKISRVE AEDLGVYFCFQGSHVPHTFGSGTKLEIK |
| 12 | Light chain variable region | DNA | atggtcagttcagcccagtttctggggcttttgctgctgtgtttccaaggta cgagatgcgacatcctcatgactcagacaccactgtcattgcccgttagcct tggcgatcaggtgtccatctcttgtcggagttcccagtctctggtgcattcc aacgggaacacctatctctgctggtatctccagaaaccaggtcaaagcccca aactgctgatttaccgtgtgagcaatcgctttagcggagtacctgacaggtt ttccggctctggaagtgggactgacttcaccctgaagatatcccgagtcgaa gctgaggatttgggagtgtacttctgcttccagggttctcacgttcctcaca cctttggctcaggcacaaagctggagattaag |
| 13 | Light chain constant region | AA | |
| 14 | Light chain constant region | DNA | cgtacggtggctgcaccatctgtcttcatcttcccgccatctgatgagcagt tgaaatctggaactgcctctgttgtgtgcctgctgaataacttctatcccag agaggccaaagtacagtggaaggtggataacgccctccaatcgggtaactcc caggagagtgtcacagagcaggacagcaaggacagcacctacagcctcagca gcaccctgacgctgagcaaagcagactacgagaaacacaaagtctacgcctg cgaagtcacccatcagggcctgagctcgcccgtcacaaagagcttcaacagg ggagagtgttag |
| 15 | Light chain CDR1 | AA | RSSQSLVHSNGNTYLC |
| 16 | Light chain CDR1 | DNA | cggagttcccagtctctggtgcattccaacgggaacacctatctctgc |
| 17 | Light chain CDR2 | AA | RVSNRFS |
| 18 | Light chain CDR2 | DNA | cgtgtgagcaatcgctttagc |
| 19 | Light chain CDR3 | AA | FQGSHVPHT |
| 20 | Light chain CDR3 | DNA | ttccagggttctcacgttcctcacacc |

**[Table 1-3]**

| TABLE 1-3 NB007-01 (heavy chain) | | | |
|---|---|---|---|
| SEQ ID NO: | Region | Type | Sequence |
| 21 | Heavy chain variable region | AA | MGWTLVFLFLLSVTAGVHSEVQLVQSGPEVVKPGASVKLSCKASGYSFTGYY MHWVKQAPGQGLEWIGRVNPNQGGTSYNKKFKVRVTITVDRSISTAYMEFSR LRSDDTVVYYCARSESNNFYAMDYWGQGTSVTVSS |
| 22 | Heavy chain variable region | DNA | atgggttggactctcgttttcctgtttctccttagcgtcactgctggcgtcc actctgaagtgcagttggtgcaatccgggccagaagttgtcaaacccggagc ctccgtcaaactgagctgtaaggcaagtggctactcctttaccgggtactac atgcattgggttaagcaggcacccggcagggcctggagtggattgggcggg taaaccctaaccaaggaggcacatcctacaacaagaagttcaaagtgcgagt gacaatcacagtggatcgctcaatctccactgcctatatggagttcagccgt ctgcgaagtgatgacaccgtggtgtattactgcgctaggagcgagtctaata atttctatgctatggactattggggtcagggaaccagcgtaaccgtgtcttc t |
| 23 | Heavy chain constant region | AA | |
| 24 | Heavy chain constant region | DNA | gctagcaccaagggcccatcggtcttccccctggcaccctcctccaagagca cctctgggggcacagcggccctgggctgcctggtcaaggactacttccccga accggtgacggtgtcgtggaactcaggcgccctgaccagcggcgtgcacacc ttcccggctgtcctacagtcctcaggactctactccctcagcagcgtggtga ccgtgccctccagcagcttgggcacccagacctacatctgcaacgtgaatca caagcccagcaacaccaaggtggacaagaaagttgagcccaaatcttgtgac aaaactcacacatgcccaccgtgcccagcacctgaactcctggggggaccgt cagtcttcctcttccccccaaaacccaaggacaccctcatgatctcccggac ccctgaggtcacatgcgtggtggtggacgtgagccacgaagaccctgaggtc aagttcaactggtacgtggacggcgtggaggtgcataatgccaagacaaagc cgcgggaggagcagtacaacagcacgtaccgtgtggtcagcgtcctcaccgt cctgcaccaggactggctgaatggcaaggagtacaagtgcaaggtctccaac aaagccctcccagcccccatcgagaaaaccatctccaaagccaaagggcagc cccgagaaccacaggtgtacaccctgcccccatcccgggatgagctgaccaa gaaccaggtcagcctgacctgcctggtcaaaggcttctatcccagcgacatc gccgtggagtgggagagcaatgggcagccggagaacaactacaagaccacgc ctcccgtgctggactccgacggctccttcttcctctacagcaagctcaccgt ggacaagagcaggtggcagcaggggaacgtcttctcatgctccgtgatgcat gaggctctgcacaaccactacacgcagaagagcctctccctgtctccgggta aatga |
| 25 | Heavy chain CDR1 | AA | GYSFTGYYMH |
| 26 | Heavy chain CDR1 | DNA | ggctactcctttaccgggtactacatgcat |
| 27 | Heavy chain CDR2 | AA | RVNPNQGGTSYNKKFKV |
| 28 | Heavy chain CDR2 | DNA | cgggtaaaccctaaccaaggaggcacatcctacaacaagaagttcaaagtg |
| 29 | Heavy chain CDR3 | AA | ARSESNNFYAMDY |
| 30 | Heavy chain CDR3 | DNA | gctaggagcgagtctaataatttctatgctatggactat |

**[Table 1-4]**

| TABLE 1-4 NB007-01 (light chain) | | | |
|---|---|---|---|
| SEQ ID NO: | Region | Type | Sequence |
| 31 | Light chain variable region | AA | MVSSAQFLGLLLLCFQGTRCDIVMTQSPLSLSVTPGQPASISCRSSQSLVHS NGNTYLCWYLQKPGQSPQLLIYRVSNKFSGVPDRFSGSGSGTDFTLKISRVE AEDVGVYYCFQGSHVPHTFGQGTKVEIK |
| 32 | Light chain variable region | DNA | atggtctcatccgcacagtttctgggcttgctcctgctctgttttcagggaa caagatgtgacatcgtcatgacccagagtccccttagcttgtcagtgactcc cggtcagccagccagtatcagctgccggagcagtcaaagcctggtccattcc aatgggaacacatatctgtgctggtatctccaaaagcctgggcagtctcccc agctgcttatttatcgcgtctccaacaaatttagtggcgttcccgaccgttt ctcaggatccggcagtggcactgatttcactctgaaaatatccagggttgag gctgaggatgtgggcgtgtactactgcttccagggctctcacgtgccacaca ccttcggtcagggaacaaaggtggagattaag |
| 33 | Light chain constant region | AA | |
| 34 | Light chain constant region | DNA | cgtacggtggctgcaccatctgtcttcatcttcccgccatctgatgagcagt tgaaatctggaactgcctctgttgtgtgcctgctgaataacttctatcccag agaggccaaagtacagtggaaggtggataacgccctccaatcgggtaactcc caggagagtgtcacagagcaggacagcaaggacagcacctacagcctcagca gcaccctgacgctgagcaaagcagactacgagaaacacaaagtctacgcctg cgaagtcacccatcagggcctgagctcgcccgtcacaaagagcttcaacagg ggagagtgttag |
| 35 | Light chain CDR1 | AA | RSSQSLVHSNGNTYLC |
| 36 | Light chain CDR1 | DNA | cggagcagtcaaagcctggtccattccaatgggaacacatatctgtgc |
| 37 | Light chain CDR2 | AA | RVSNKFS |
| 38 | Light chain CDR2 | DNA | cgcgtctccaacaaatttagt |
| 39 | Light chain CDR3 | AA | FQGSHVPHT |
| 40 | Light chain CDR3 | DNA | ttccagggctctcacgtgccacacacc |

**[Table 1-5]**

| TABLE 1-5 NB007-02 (heavy chain) | | | |
|---|---|---|---|
| SEQ ID NO: | Region | Type | Sequence |
| 41 | Heavy chain variable region | AA | MGWTLVFLFLLSVTAGVHSEVQLVQSGPEVVKPGASVKLSCKASGYSFTGYY MHWVKQAPGQGLEWIGRVNPNQGGTSYNKKFKVRVTLTVDRSISTAYMEFSR LRSDDTVVYYCARSESNNFYAMDYWGQGTSVTVSS |
| 42 | Heavy chain variable region | DNA | atgggctggactttggtgttcctctttctgctgtctgtcactgctggtgtac actccgaagtgcagcttgtacagtcaggacccgaagtggtgaaaccaggtgc tagtgtcaagctcagctgcaaagcaagcgggtatagctttaccggctattac atgcactgggtgaaacaggcacctggtcagggactggaatggattgggaggg ttaatcccaatcagggaggcacatcctacaacaagaagttcaaggtgcgcgt gacactgacagtggatcggtccatttcaaccgcctacatggagttttctcgc ctccgttccgacgataccgtggtctactactgtgcaagatcagagtccaaca acttctatgccatggactattggggacaaggcacaagtgtaaccgtgtcatc t |
| 43 | Heavy chain constant region | AA | |
| 44 | Heavy chain constant region | DNA | gctagcaccaagggcccatcggtcttccccctggcaccctcctccaagagca cctctgggggcacagcggccctgggctgcctggtcaaggactacttccccga accggtgacggtgtcgtggaactcaggcgccctgaccagcggcgtgcacacc ttcccggctgtcctacagtcctcaggactctactccctcagcagcgtggtga ccgtgccctccagcagcttgggcacccagacctacatctgcaacgtgaatca caagcccagcaacaccaaggtggacaagaaagttgagcccaaatcttgtgac aaaactcacacatgcccaccgtgcccagcacctgaactcctggggggaccgt cagtcttcctcttccccccaaaacccaaggacaccctcatgatctcccggac ccctgaggtcacatgcgtggtggtggacgtgagccacgaagaccctgaggtc aagttcaactggtacgtggacggcgtggaggtgcataatgccaagacaaagc cgcgggaggagcagtacaacagcacgtaccgtgtggtcagcgtcctcaccgt cctgcaccaggactggctgaatggcaaggagtacaagtgcaaggtctccaac aaagccctcccagcccccatcgagaaaaccatctccaaagccaaagggcagc cccgagaaccacaggtgtacaccctgcccccatcccgggatgagctgaccaa gaaccaggtcagcctgacctgcctggtcaaaggcttctatcccagcgacatc gccgtggagtgggagagcaatgggcagccggagaacaactacaagaccacgc ctcccgtgctggactccgacggctccttcttcctctacagcaagctcaccgt ggacaagagcaggtggcagcaggggaacgtcttctcatgctccgtgatgcat gaggctctgcacaaccactacacgcagaagagcctctccctgtctccgggta aatga |
| 45 | Heavy chain CDR1 | AA | GYSFTGYYMH |
| 46 | Heavy chain CDR1 | DNA | gggtatagctttaccggctattacatgcac |
| 47 | Heavy chain CDR2 | AA | RVNPNQGGTSYNKKFKV |
| 48 | Heavy chain CDR2 | DNA | agggttaatcccaatcagggaggcacatcctacaacaagaagttcaaggtg |
| 49 | Heavy chain CDR3 | AA | ARSESNNFYAMDY |
| 50 | Heavy chain CDR3 | DNA | gcaagatcagagtccaacaacttctatgccatggactat |

**[Table 1-6]**

| TABLE 1-6 NB007-02 (light chain) | | | |
|---|---|---|---|
| SEQ ID NO: | Region | Type | Sequence |
| 51 | Light chain variable region | AA | MVSSAQFLGLLLLCFQGTRCDIVMTQSPLSLSVTPGQPASISCRSSQSLVHS NGNTYLCWYLQKPGQSPQLLIYRVSNRFSGVPDRFSGSGSGTDFTLKISRVE AEDVGVYYCFQGSHVPHTFGQGTKVEIK |
| 52 | Light chain variable region | DNA | atggtcagttcagcccagtttctggggcttttgctgctgtgtttccaaggta cgagatgcgacatcgtcatgactcagtctccactgtcattgagcgttactcc aggtcaaccagcctccatctcttgtcggagttcccagtctctggtgcattcc aacgggaacacctatctctgctggtatctccagaaaccaggtcaaagccccc agctgctgatttaccgtgtgagcaatcgctttagcggagtacctgacaggtt ttccggctctggaagtgggactgacttcaccctgaagatatcccgagtcgaa gctgaggatgttggagtgtactactgcttccagggttctcacgttcctcaca cctttggccagggcacaaaggtggagattaag |
| 53 | Light chain constant region | AA | |
| 54 | Light chain constant region | DNA | cgtacggtggctgcaccatctgtcttcatcttcccgccatctgatgagcagt tgaaatctggaactgcctctgttgtgtgcctgctgaataacttctatcccag agaggccaaagtacagtggaaggtggataacgccctccaatcgggtaactcc caggagagtgtcacagagcaggacagcaaggacagcacctacagcctcagca gcaccctgacgctgagcaaagcagactacgagaaacacaaagtctacgcctg cgaagtcacccatcagggcctgagctcgcccgtcacaaagagcttcaacagg ggagagtgttag |
| 55 | Light chain CDR1 | AA | RSSQSLVHSNGNTYLC |
| 56 | Light chain CDR1 | DNA | cggagttcccagtctctggtgcattccaacgggaacacctatctctgc |
| 57 | Light chain CDR2 | AA | RVSNRFS |
| 58 | Light chain CDR2 | DNA | cgtgtgagcaatcgctttagc |
| 59 | Light chain CDR3 | AA | FQGSHVPHT |
| 60 | Light chain CDR3 | DNA | ttccagggttctcacgttcctcacacc |

**[Table 1-7]**

| TABLE 1-7 NB007-03 (heavy chain) | | | |
|---|---|---|---|
| SEQ ID NO: | Region | Type | Sequence |
| 61 | Heavy chain variable region | AA | MGWTLVFLFLLSVTAGVHSEVQLVQSGPEVVKPGASVKLSCKASGYSFTGYY MHWVKQAPGQGLEWIGRVNPNNGGTSYNKKFKVRVTLTVDRSISTAYMEFSR LRSDDTVVYYCARSESNNFYAMDYWGQGTSVTVSS |
| 62 | Heavy chain variable region | DNA | atgggctggactttggtgttcctctttctgctgtctgtcactgctggtgtac actccgaagtgcagcttgtacagtcaggacccgaagtggtgaaaccaggtgc tagtgtcaagctcagctgcaaagcaagcgggtatagctttaccggctattac atgcactgggtgaaacaggcacctggtcagggactggaatggattgggaggg ttaatcccaataatggaggcacatcctacaacaagaagttcaaggtgcgcgt gacactgacagtggatcggtccatttcaaccgcctacatggagttttctcgc ctccgttccgacgataccgtggtctactactgtgcaagatcagagtccaaca acttctatgccatggactattggggacaaggcacaagtgtaaccgtgtcatc t |
| 63 | Heavy chain constant region | AA | |
| 64 | Heavy chain constant region | DNA | gctagcaccaagggcccatcggtcttccccctggcaccctcctccaagagca cctctgggggcacagcggccctgggctgcctggtcaaggactacttccccga accggtgacggtgtcgtggaactcaggcgccctgaccagcggcgtgcacacc ttcccggctgtcctacagtcctcaggactctactccctcagcagcgtggtga ccgtgccctccagcagcttgggcacccagacctacatctgcaacgtgaatca caagcccagcaacaccaaggtggacaagaaagttgagcccaaatcttgtgac aaaactcacacatgcccaccgtgcccagcacctgaactcctggggggaccgt cagtcttcctcttccccccaaaacccaaggacaccctcatgatctcccggac ccctgaggtcacatgcgtggtggtggacgtgagccacgaagaccctgaggtc aagttcaactggtacgtggacggcgtggaggtgcataatgccaagacaaagc cgcgggaggagcagtacaacagcacgtaccgtgtggtcagcgtcctcaccgt cctgcaccaggactggctgaatggcaaggagtacaagtgcaaggtctccaac aaagccctcccagcccccatcgagaaaaccatctccaaagccaaagggcagc cccgagaaccacaggtgtacaccctgcccccatcccgggatgagctgaccaa gaaccaggtcagcctgacctgcctggtcaaaggcttctatcccagcgacatc gccgtggagtgggagagcaatgggcagccggagaacaactacaagaccacgc ctcccgtgctggactccgacggctccttcttcctctacagcaagctcaccgt ggacaagagcaggtggcagcaggggaacgtcttctcatgctccgtgatgcat gaggctctgcacaaccactacacgcagaagagcctctccctgtctccgggta aatga |
| 65 | Heavy chain CDR1 | AA | GYSFTGYYMH |
| 66 | Heavy chain CDR1 | DNA | gggtatagctttaccggctattacatgcac |
| 67 | Heavy chain CDR2 | AA | RVNPNNGGTSYNKKFKV |
| 68 | Heavy chain CDR2 | DNA | agggttaatcccaataatggaggcacatcctacaacaagaagttcaaggtg |
| 69 | Heavy chain CDR3 | AA | ARSESNNFYAMDY |
| 70 | Heavy chain CDR3 | DNA | gcaagatcagagtccaacaacttctatgccatggactat |

**[Table 1-8]**

| TABLE 1-8 NB007-03 (light chain) | | | |
|---|---|---|---|
| SEQ ID NO: | Region | Type | Sequence |
| 71 | Light chain variable region | AA | MVSSAQFLGLLLLCFQGTRCDIVMTQTPLSLSVTLGQPASISCRSSQSLVHS NGNTYLCWYLQKPGQSPQLLIYRVSNRFSGVPDRFSGSGSGTDFTLKISRVE AEDVGVYYCFQGSHVPHTFGSGTKVEIK |
| 72 | Light chain variable region | DNA | atggtcagttcagcccagtttctggggcttttgctgctgtgtttccaaggta cgagatgcgacatcgtcatgactcagacaccactgtcattgagcgttactct cggtcaaccagcctccatctcttgtcggagttcccagtctctggtgcattcc aacgggaacacctatctctgctggtatctccagaaaccaggtcaaagccccc agctgctgatttaccgtgtgagcaatcgctttagcggagtacctgacaggtt ttccggctctggaagtgggactgacttcaccctgaagatatcccgagtcgaa gctgaggatgttggagtgtactactgcttccagggttctcacgttcctcaca cctttggcagcggcacaaaggtggagattaag |
| 73 | Light chain constant region | AA | |
| 74 | Light chain constant region | DNA | cgtacggtggctgcaccatctgtcttcatcttcccgccatctgatgagcagt tgaaatctggaactgcctctgttgtgtgcctgctgaataacttctatcccag agaggccaaagtacagtggaaggtggataacgccctccaatcgggtaactcc caggagagtgtcacagagcaggacagcaaggacagcacctacagcctcagca gcaccctgacgctgagcaaagcagactacgagaaacacaaagtctacgcctg cgaagtcacccatcagggcctgagctcgcccgtcacaaagagcttcaacagg ggagagtgttag |
| 75 | Light chain CDR1 | AA | RSSQSLVHSNGNTYLC |
| 76 | Light chain CDR1 | DNA | cggagttcccagtctctggtgcattccaacgggaacacctatctctgc |
| 77 | Light chain CDR2 | AA | RVSNRFS |
| 78 | Light chain CDR2 | DNA | cgtgtgagcaatcgctttagc |
| 79 | Light chain CDR3 | AA | FQGSHVPHT |
| 80 | Light chain CDR3 | DNA | ttccagggttctcacgttcctcacacc |

### [Example 2] Efficacy of Humanized Anti-CCR7 Antibody

A human CCR7 gene-transfected cell (described in WO 2012/043533 and JP Patent No. 5315495) was seeded into a 96-well microplate at an initial cell concentration of 2×10⁴ cells/100 µL per well and cultured for 2 days. After 2 days, the culture medium was exchanged with a solution containing 3 µM of Cal-520 (AAT Bioquest, Inc.), 0.05% of Pluronic-F127, and 2.5 mM of probenecid (Invitrogen Corporation). After 1 hour, humanized anti-CCR7 antibody NB007-01, NB007-02, or NB007-03 was added to each well at a concentration within a range of 10⁻⁶ to 10⁻¹⁰ M. As a positive control, the chimeric antibody VH0/VL0 was also added to other wells at a concentration within a range of 10⁻⁶ to 10⁻¹⁰ M.

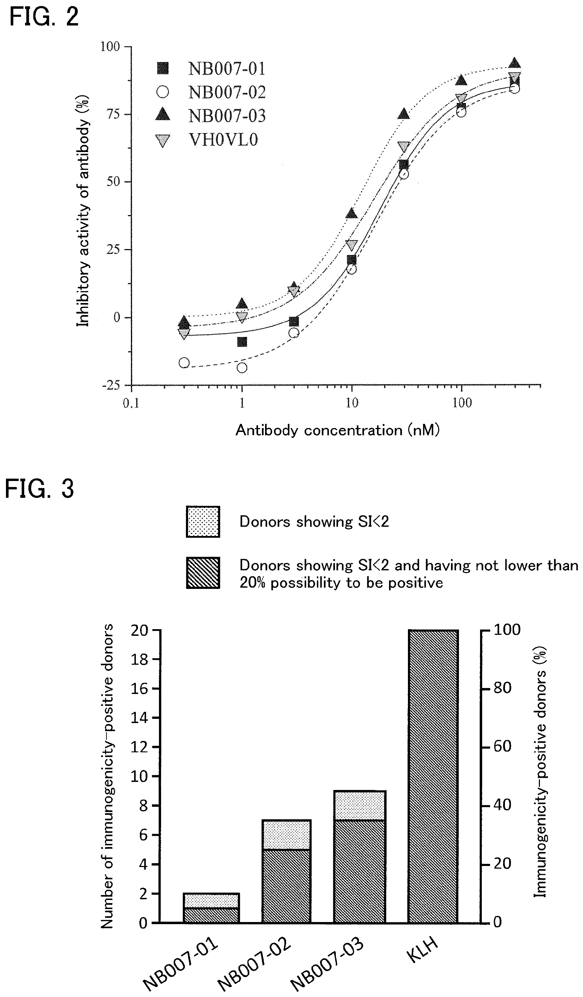

After 15 minutes, each cell was stimulated with 5×10⁻⁸ M of CCL21 (R&D Systems, Inc.). At this time, an intracellular Ca²⁺ concentration was measured using a Ca²⁺ signaling measurement device (FDSS/µCELL; Hamamatsu Photonics K.K.) to analyze an inhibitory activity of each additive (antibody) on the intracellular Ca²⁺ signaling. The results are presented in Fig. 2 and Table 2. Fig. 2 is a graph presenting a relationship between an inhibitory activity of the antibody on the intracellular Ca²⁺ signaling and an antibody concentration. The inhibitory activity was calculated as a relative value based on a standardization in which an inhibition rate in a case of "no antibody and any ligand" was defined as 0% and an inhibition rate in a case of "no antibody and no ligand" was defined as 100%. Table 2 presents 50% inhibitory concentrations (IC50) of each antibody, calculated from the analysis results of Fig. 2.

As presented in Fig. 2, VH0/VL0, NB007-01, NB007-02, and NB007-03 all inhibited the intracellular Ca²⁺ signaling in a concentration-dependent manner. This indicates that the inhibition of the binding between CCL21 and human CCR7 by each additive results in inhibition of the intracellular Ca²⁺ signaling. The IC50 values were 16.2 nM for VH0/VL0, 18.2 nM for NB007-01, 16.4 nM for NB007-02, and 12.5 nM for NB007-03 (Table 2). The above results indicated that the humanized anti-CCR7 antibodies obtained all had a CCR7 inhibitory activity equivalent to that of the chimeric antibody VH0/VL0.

### [Table 2]

**TABLE 2**

| | VH0/VL0 | NB007-01 | NB007-02 | NB007-03 |
|---|---|---|---|---|
| IC50 (nM) | 16.2 | 18.2 | 16.4 | 12.5 |

### [Example 3] Immunogenicity of Humanized Anti-CCR7 Antibody

The immunogenicities of NB007-01, NB007-02, and NB007-03 on human peripheral blood mononuclear cells (PBMC) were evaluated.

### (1) Preparation of Human PBMC

From whole blood collected from healthy volunteers, human PBMCs were separated by a density gradient method, to which human AB serum or fetal bovine serum and 10% dimethylsulfoxide were added, and the mixture was cryopreserved. The human PBMCs were stored at -180°C until use.

### (2) DC-T Cell Assay

Monocytes were separated from human PBMCs by magnetic separation, and cultured in a dendritic cell (DC) medium containing interleukin-4 (IL-4) and a granulocyte-macrophage colony-stimulating factor (GM-CSF) for 5 days to differentiate the monocytes into immature DCs (iDCs). The iDCs were collected and seeded into a cell culture plate, to which the test substance NB007-01, NB007-02, or NB007-03 was added, and furthermore the iDCs were cultured overnight in a DC medium containing interleukin-1β (IL-1β) and tumor necrosis factor α (TNF-α) to differentiate the iDCs into mature DCs. Then, NB007-01, NB007-02, NB007-03, and a cytokine cocktail were removed by washing.

From human PBMCs, CD4+ T cells were isolated by magnetic separation using negative selection (StemCells: EasySep^{™} Human CD4+ T Cell Enrichment Kit; 19052). The CD4+ T cells and mature DCs were co-cultured in a serum-supplemented medium for 6 days. To the co-culture medium of mature DCs and T cells, 5-ethynyl-2'-deoxyuridine (EdU) was added. After 16 hours, viable and dead cells were separated by fluorescence labeling, and further stained with CD3 and CD4 which are surface markers for T cells. Then, the cells were immobilized and subjected to membrane permeation, and sites where EdU had been incorporated were marked by staining with a fluorescent azide and analyzed by a flow cytometry (LSR Fortessa, Becton, Dickinson and Company). Proliferative Th cells were defined as CD3-positive, CD4-positive, and EdU-positive cells, and subjected to analysis using FlowLogic software.

### (3) Statistic Analysis

The positive reactions of the test substances (NB007-01, NB007-02, or NB007-03) to the donors were evaluated by calculating a stimulation index (SI) defined as a ratio of an average reactivity of the test substances to an average reactivity of the controls or reference substances. For the control or reference substances, Keyhole limpet hemocyanin (KLH) was used. The number of the proliferative Th cells per well, or the number of the CD3-positive, CD4-positive, and EdU-positive cells was evaluated based on the signaling response. Donors that satisfied a condition of SI>2 were defined as positive based on the calculated SI.

Furthermore, an evaluation using a statistic equivalence test was also conducted to reduce the risk of false-negative. Herein, a distribution free resampling (DFR) method reported by Moody et al. was used.

The results are presented in Table 3 and Fig. 3. The number of donors positive for immunogenicity of NB007-01 was statistically 2 out of 20, indicating that NB007-01 was less immunogenic than the control substance. Similarly, the number of donors positive for immunogenicity of NB007-02 was statistically 7 out of 20, indicating that NB007-02 was less immunogenic than the control substance. Similarly, the number of donors positive for immunogenicity of NB007-03 was statistically 9 out of 20, indicating that NB007-03 was less immunogenic than the control substance. Above all, the immunogenicity of NB007-01 with the modified CDR was improved more than expected compared to NB007-03 with the unmodified CDR.

### [Table 3]

**TABLE 3**

| Test substance | Number of donors showing SI>2 | Number of donors showing SI<2 but having statistically not lower than 20% possibility to be positive | Number of positive donors |
|---|---|---|---|
| KLH | 20 | 0 | 20 |
| NB007-01 | 1 | 1 | 2 |
| NB007-02 | 5 | 2 | 7 |
| NB007-03 | 7 | 2 | 9 |

### [Example 4] Evaluation of Cell Binding Property by Flow Cytometry

Binding of the antibody to hCCR7 gene-transfected cells and binding of the antibody to human lymphoma cell line MJ cells derived from T-cell lymphoma were evaluated by using NB007-01 labeled with a fluorescent dye Allophycocyanin (APC). Binding of the antibody to human lymphoma cell line Granta-519 cells derived from B cell lymphoma was evaluated by using unlabeled NB007-01 and using Alexa Fluor 647-labeled anti-human IgG antibody (Thermo Fisher Scientific, Inc.) as the secondary antibody for detection. When the antibody was labeled with APC, an APC Conjugation Kit-Lighting-Link (Abcam PLC) was used. To 100 µL of antibody solution prepared by diluting the antibody with PBS so that the antibody concentration was 1 mg/mL, 10 µL of modifier reagent was added, and this mixture was reacted with APC Conjugation Mix. This mixture was stored in a dark place at room temperature for 3 hours, and then 10 µL of quencher reagent was added to the mixture, which was used as a fluorescent-labeled antibody.

The hCCR7 gene-transfected cells were detached from the culture dish using Cell Dissociation Buffer, enzyme-free, Hanks' Balanced Salt Solution (Thermo Fisher Scientific, Inc.), and washed with PBS. On the other hand, required amounts of Granta-519 cells and MJ cells, which are suspension cells, were separately collected and washed with PBS. To a cell suspension prepared by suspending the cells in PBS so that the cell concentration was 1×10⁷ cells/mL, an equal volume of Goat serum (Thermo Fisher Scientific, Inc.) was added, and the mixture was allowed to stand at 4°C for 30 minutes to block the cells. The mixture was centrifuged at 200 g for 5 minutes, the supernatant was removed, the cells were suspended in a fluorescence-activated cell sorting (FACS) buffer (PBS containing 1% FBS) so that the cell concentration was 4×10⁶ cells/mL, and 50µL of the suspension was dispensed into each well of a 96-well plate. The fluorescent-labeled NB007-01 antibody or unlabeled NB007-01 diluted with FACS buffer was added to the wells, and allowed to stand at 4°C for 1 hour. The cells were collected by centrifugation at 200 g for 5 minutes, and washed with 100 µL of FACS buffer. This washing process was repeated twice. The hCCR7 gene-transfected cells and MJ cells that had been brought into contact with the labeled NB007-01 were suspended in 100 µL of FACS buffer, and then the binding between the cells and the labeled antibody was measured using a flow cytometer CytoFLEX (Beckman Coulter, Inc.). The Granta-519 cells that had been brought into contact with the unlabeled NB007-01 were suspended in 50 µL of Alexa Fluor 647-labeled anti-human IgG antibody dilution diluted 800-fold with FACS buffer and allowed to stand at 4°C for 1 hour. The cells were washed twice and then suspended in 100 µL of FACS buffer, and the binding of the cells to the antibody was measured using the flow cytometer.

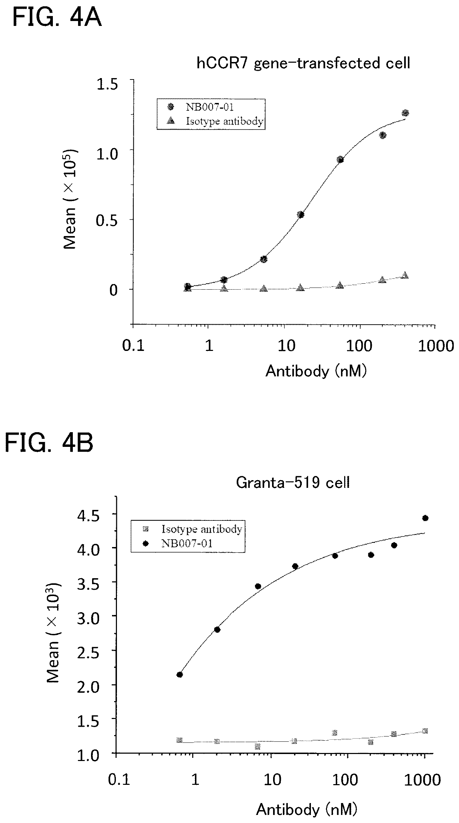

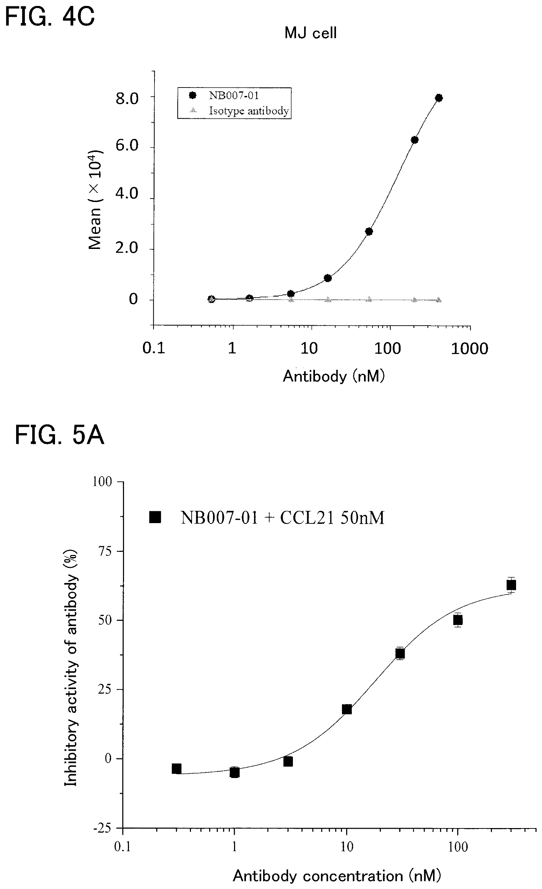

Fig. 4A to Fig. 4C present graphs in which the concentration of the antibody is plotted on the horizontal axis and the geometric average of the fluorescence intensity in the FACS histogram is plotted on the vertical axis. From these graphs, the specific bindings of NB007-01 to hCCR7 gene-transfected cells (Fig. 4A), Granta-519 cells (Fig. 4B), and MJ cells (Fig. 4C) were confirmed.

### [Example 5] Evaluation of NB007-01 Function by Measurement of Intracellular Ca²⁺ Signaling

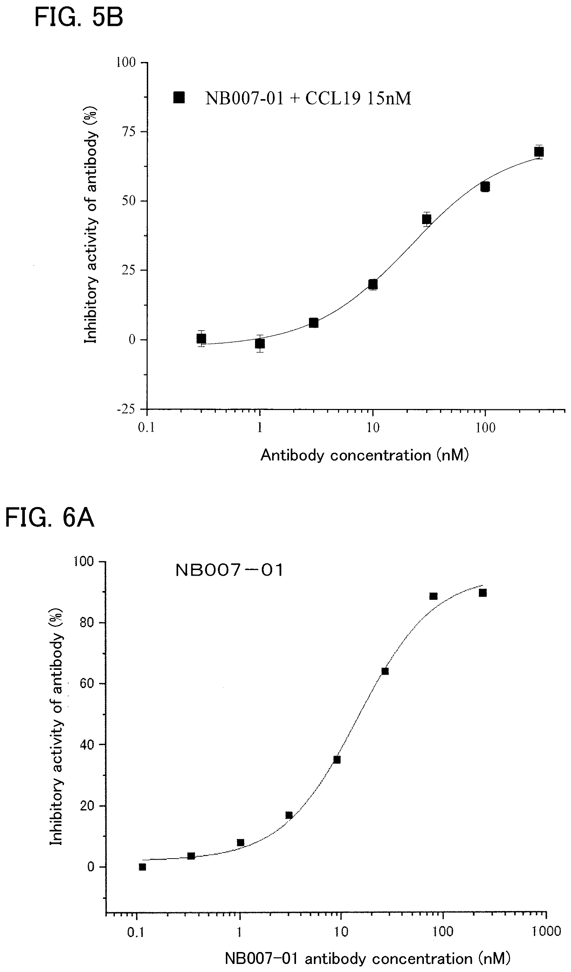

In the same procedure as in Example 2, hCCR7 gene-transfected cells were seeded into a 96-well microplate and cultured for 2 days. The culture medium was exchanged with a solution containing 3 µM of Cal-520 (AAT Bioquest, Inc.), 0.05% of Pluronic-F127, and 2.5 mM of probenecid (Invitrogen Corporation), and after 1 hour, NB007-01 at a concentration within a range of 10⁻⁶ to 10⁻¹⁰ M was added to each well. After 15 minutes, each cell was stimulated with 5×10⁻⁸ M of CCL21 (R&D Systems, Inc.) or 1.5×10⁻⁸ M of CCL19 (R&D Systems, Inc.). An inhibitory activity of NB007-01 on the intracellular Ca²⁺ signaling was analyzed using a Ca²⁺ signaling measurement device. The results are presented in Fig. 5A and Fig. 5B. Fig. 5A and Fig. 5B are graphs presenting a relationship between the inhibitory activity of the antibody on the intracellular Ca²⁺ signaling and an antibody concentration, wherein the cases that the cells are stimulated with CCL21 is in Fig. 5A and with CCL19 is in Fig. 5B. The inhibitory activity was calculated as a relative value based on a standardization in which an inhibition rate in a case of "no antibody and any CCL21 or CCL19" was defined as 0% and an inhibition rate in a case of "no antibody and no CCL21 or CCL19" was defined as 100%.

As presented in Fig. 5A and Fig. 5B, NB007-01 inhibited the CCL21 and CCL19-induced intracellular Ca²⁺ signaling in a concentration-dependent manner. The IC50 values were 17.8 nM for the CCL 21 stimulation, and 21.4 nM for the CCL19 stimulation respectively. The above results indicated that NB007-01 inhibited the CCL21 and CCL19-induced intracellular Ca²⁺ signaling.

### [Example 6] Evaluation of Inhibitory Activity on Intracellular Ca²⁺ Signaling Using Human Lymphoma Cell Line

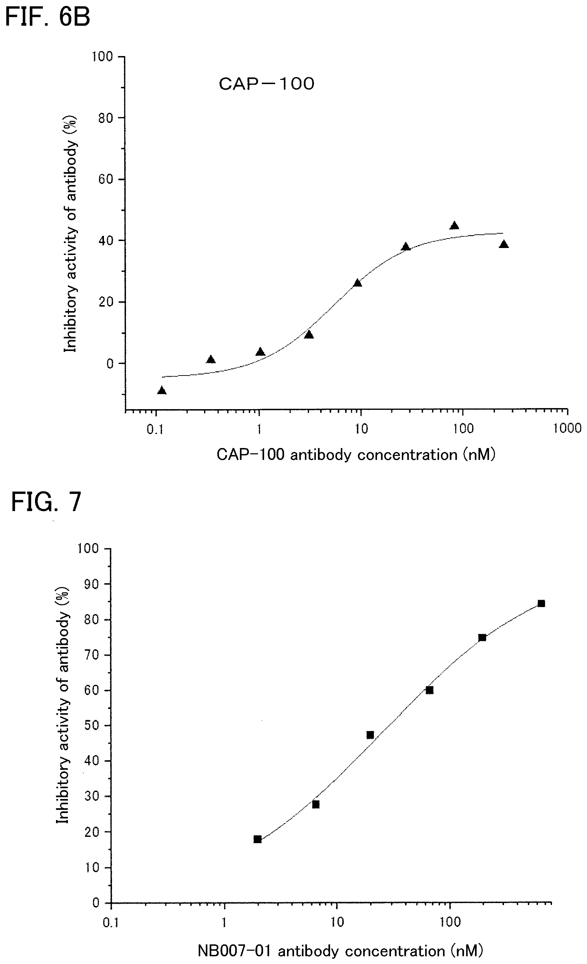

MJ cells were washed with Hanks' Balanced Salts Solution (HBSS) (containing CaCl₂ and MgCl₂)+0.1% BSA assay buffer, then mixed with a solution containing 1 µM of Cal-520 (AAT Bioquest, Inc.) and 0.05% of Pluronic-F127 so that the cell concentration was 1.16 ×10⁶ cells/mL, and incubated at 37°C. After 45 minutes, the cells were washed twice with the assay buffer, the cell concentration was adjusted to 1×10⁶ cells/mL, and 80 µL of the cell suspension was seeded into each well of a 96-well black poly-D-lysine (PDL)-coated microplate (CELLCOAT, Greiner Bio-One International GmbH). NB007-01 or CAP-100 serially diluted by 3 times was added to each well at 8 concentration points within a concentration range of 250 nM to 0.11 nM, and incubated for 15 minutes. The assay plate was placed in a Ca²⁺ signaling analyzer (FDSSµCELL; Hamamatsu Photonics K.K.), and the cells were stimulated with 20 nM of CCL21 (R&D Systems, Inc.), and, at the time of this stimulation, the inhibitory activity of each antibody on the intracellular Ca²⁺ signaling was analyzed. The result of NB007-01 is presented in Fig. 6A, and the result of CAP-100 is presented in Fig. 6B. As presented in Fig. 6A, NB007-01 inhibited the CCL21-induced intracellular Ca²⁺ signaling in a concentration-dependent manner, with a half maximal inhibitory concentration (IC50) of 14.8 nM. On the other hand, as presented in Fig. 6B, CAP-100 inhibited the CCL21-induced intracellular Ca²⁺ signaling in a concentration-dependent manner, but the maximum inhibition rate was approximately 50%.

### [Example 7] Evaluation of Cell Migration Inhibiting Activity Using Human Lymphoma Cell Line

Granta-519 cells were stained with CytoRed (Dojindo Laboratories Co., Ltd..), then washed twice with RPMI+0.5% BSA. NB007-01 (0.3 µg to 100 µg/mL) or a negative control human IgG1 antibody (Icosagen) (1 µg/mL or 100 µg/mL) was added to a cell suspension at 4×10⁶ cells/mL, and allowed to stand at 37°C for 30 minutes.

To wells of a 3.0 µm polycarbonate membrane (Corning Inc.) receiver plate in insert-integrated 96-well Transwell, 50 ng/mL of CCL19 was added. The insert of Transwell was placed on the receiver plate, then 50 µL of the cell suspension described above was seeded into the insert and allowed to stand at 37°C. After 4 hours, the insert was removed, each well was photographed in three visual fields using a fluorescent 4-power lens, and migrating cells were counted. The inhibitory activity was calculated as a relative value based on a standardization in which an inhibition rate in a case of "no antibody and any CCL19" was defined as 0% and an inhibition rate in a case of "no antibody and no CCL19" was defined as 100%. Fig. 7 presents the inhibitory effect of NB007-01 on the CCL19-dependent cell migration. The IC50 of NB007-01 was 25.5 nM. The inhibition rates of human IgG1 antibody as the negative control at 1 µg/mL and 100 µg/mL were 3.0% and 3.3% respectively.

### [Example 8] Preparation of Defucosylated NB007-01

As the method for defucosylating the antibody, GlymaxX method (WO 2011/035884, JP Patent No. 5746183) was used. In the procedure for preparing NB007-01 using QMCF method in Example 1, defucosylated NB007-01 was prepared using a CHO cell line (CHOEBNALT85-RMD C4) modified in GlymaxX method as the CHO cell used for expression of antibody genes. The antibody was purified.

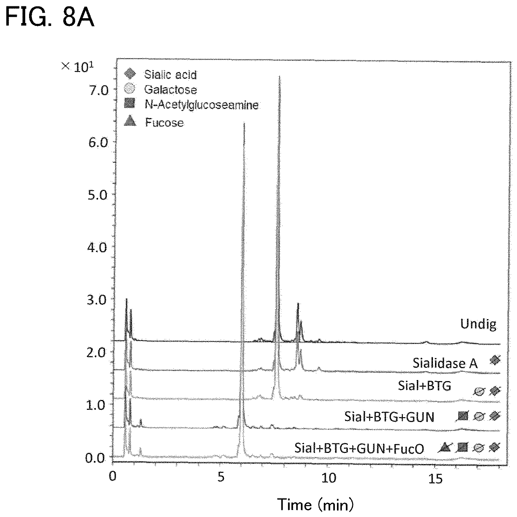

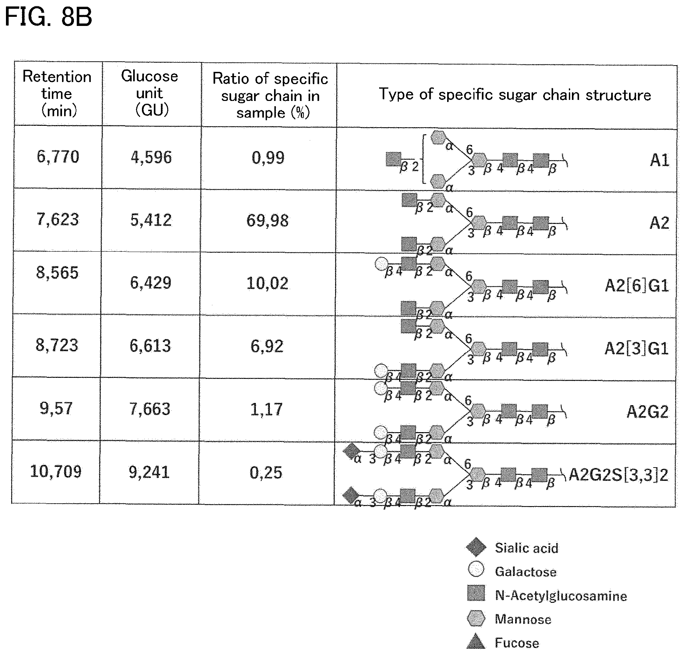

N-type sugar chains were cleaved out from the purified antibody and labeled according to an manual using AdvanceBio Gly-X-N-glycan prep with InstantPC Kit (Agilent Technologies, Inc.), sialidase (AdvanceBio Sialidase A) (GK80040, Agilent Technologies, Inc.), β1-3,4 galactosidase (BTG) (P0746S, New England Biolabs.), β-N-acetylglucosaminidase S (GUH) (P0744, New England Biolabs.), and α1-2,4,6 fucosidase O (FucO) (P0749, New England Biolabs.). The labeled N-type sugar chains were subjected to a high-performance liquid chromatography (HPLC) (Agilent 1260 Infinity II) analysis using AdvanceBio Glycan 2.7 µm column (Agilent Technologies, Inc.) according to a manual. The results of the HPLC chromatogram are presented in Fig. 8A, and percentages of each sugar chain type calculated from the chromatogram are presented in Fig. 8B. In Fig. 8A, "Undig" indicates "undigested", "Sialidase A" indicates "digested with sialidase", "Sia1+BTG" indicates "digested with sialidase+BTG", "Sial+BTG+GUN" indicates "digested with sialidase+BTG+GUN", and "Sial+BTG+GUN+FucO" indicates "digested with sialidase+BTG+GUN+FucO", respectively. As presented in Fig. 8A and Fig. 8B, most of the separated peaks were assigned to specific sugar chains based on their relative retention times (glucose unit), and accounted for 89% of the peak areas of all samples. In the FucO cleavage experiment, there was almost no change in the peaks, and 99% or higher of the identified sugar chain types were defucosylated.

### [Example 9] Evaluation of Cytotoxicity Function of Defucosylated NB007-01

### (1) ADCC Reporter Bioassay

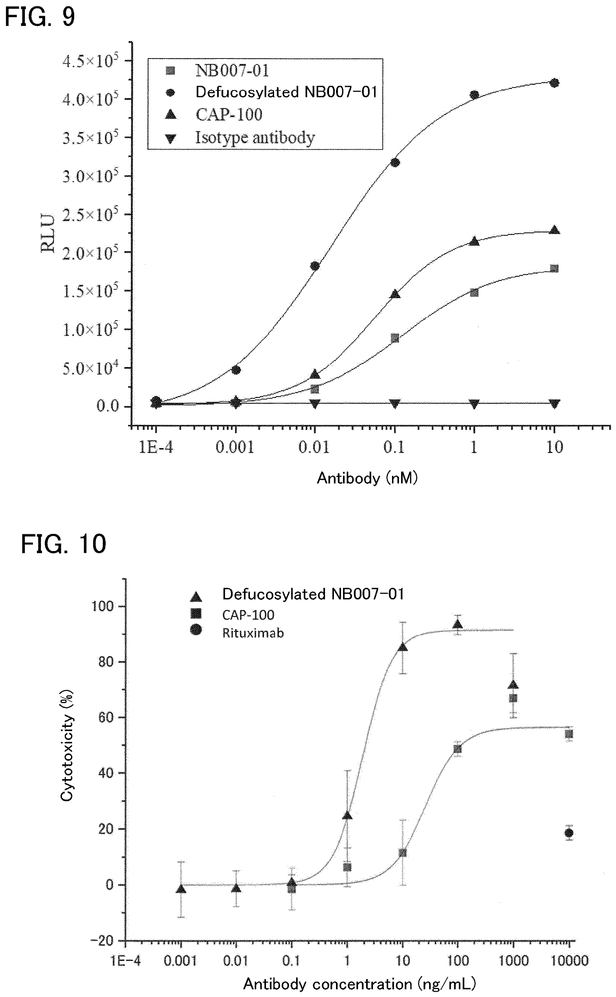

The ADCC function of the defucosylated antibody was evaluated using an ADCC reporter bioassay, V Variant (Promega). First, 1.4 mL of Low IgG Serum provided in the kit was mixed with 33.6 mL of RPMI 1640 Medium to prepare an ADCC assay buffer, into which Granta-519 cells were suspended at a cell density of 5×10⁵ cells/mL. A dilution solution of the antibody as the test substance was prepared using the ADCC assay buffer. As the test substances, NB007-01, defucosylated NB007-01, CAP-100, and human IgG (manufactured by Icosagen) as a negative control were used. Then, 630 µL of ADCC Bioassay Effector Cells provided in the kit were added to 3.6 mL of the ADCC Assay Buffer to prepare an effector cell suspension. Into each well of Culture plate-96 (PerkinElmer, Inc.), 25 µL each of antibody dilution, Granta-519 cell suspension, and effector cell suspension were dispensed, and allowed to stand in 5% CO₂ environment at 37°C for 6 hours. Luciferase Assay Substrate provided in the kit was dissolved in Luciferase Assay Buffer, and 75 µL of this solution was added to each well of the plate. The plate was allowed to stand in a dark place at room temperature for 15 minutes, then a relative luminescence intensity (Relative Luminescence Units; RLU) was measured using a microplate reader ARVO (PerkinElmer, Inc.). Fig. 9 is a graph in which the antibody concentration is plotted on the horizontal axis and the RLU is plotted on the vertical axis. The RLU is an indicator for activation of the effector cells, and this bioassay is a functionality measurement method based on the ADCC mechanism. As presented in Fig. 9, the defucosylated NB007-01 (EC50=16.1 pM) had an ADCC function considerably improved compared to NB007-01 (EC50=123 pM), and was superior to CAP-100 (EC50=57.1 pM).

### (2) Method for Measuring Cytotoxicity Activity Using Human PBMC

### (a) Preparation of Human PBMC

Frozen human PBMCs were thawed in a water bath at 37°C. The PBMCs were washed with a complete medium, then resuspended in the complete medium so that the cell concentration was 2×10⁶ cells/mL, and cultured under 5% CO₂ at 37°C overnight.

### (b) Pretreatment of Granta-519 Cell

Granta-519 cells were prepared in an ADCC medium so that a viable cell concentration was 3×10⁴ cells/80 µL, and seeded into each well of a 96-well round-bottom plate. Then, 20µL of the test substance diluted with the ADCC medium was further added to each well and allowed to stand at room temperature for 30 minutes. As the test substances, defucosylated NB007-01 (1000 to 0.0001 ng/mL), CAP-100 (10000 to 0.1 ng/mL), and Rituximab (10000 ng/mL, F. Hoffmann-La Roche, Ltd.) were used.

### (c) Measurement of ADCC Activity

Human PBMCs were prepared as effector cells (E), and Granta-519 cells were prepared as target cells (T) so that a ratio of E:T was 50:1. That means, human PBMCs in (a) were washed with an ADCC medium, then the cell concentration was adjusted with the ADCC medium, and the human PBMCs at 1.5×10⁶ cells/100 µL were added to each well in (b). The cells were allowed to stand at 37°C for 5 hours, then centrifuged at 250×g for 4 minutes, and 50µL of the supernatant was collected into each well of a clear flat-bottom 96-well plate. Incidentally, 45 minutes before the collection of the supernatant, a control for the maximum cytotoxicity condition, added with 20 µL of lysis solution (G1780, CytoTox 96 Non-Radioactive Cytotoxicity Assay Kit, Promega) was prepared.

Subsequently, 50 µL of substrate mixture (G1780, CytoTox 96 Non-Radioactive Cytotoxicity Assay Kit, Promega) was added to each well, and mixed in the plate for 30 seconds. Then the mixture was allowed to stand at room temperature for 30 minutes. Subsequently, 50 µL of stop reagent (G1780, CytoTox 96 Non-Radioactive Cytotoxicity Assay kit, Promega) was added to each well, mixed for 30 minutes, and an absorbance at 490 nm was measured. The cytotoxicity activity (%) was calculated as a relative value based on a standardization in which the maximum cytotoxicity condition was defined as 100% and the condition without the test substance was defined as 0%. Fig. 10 presents a graph in which the calculated values are plotted. In Fig. 10, "aNB007" refers to the defucosylated NB007-01.

Also in the results of this study, the defucosylated NB007-01 (EC50=1.9 ng/mL, 12.6 pM in terms of IgG) showed a cytotoxicity activity superior to CAP-100 (EC50=26.3 ng/mL, 175 pM in terms of IgG). Rituximab that has been clinically used because of its antitumor effect based on the ADCC activity showed only 18.6% cytotoxicity activity at a concentration of 10000 ng/mL, suggesting the superiority in the tumor treatment effect of defucosylated NB007-01.

### [Example 10] Antitumor Activity of Humanized Anti-CCR7 Antibody

The antitumor activities of NB007-01, defucosylated NB007-01, and CAP-100 were evaluated in tumor models transplanted with Granta-519 cells. Female CB17/SCID mice aged 6 to 8 weeks were used as model animals.

Granta-519 cells were cultured in a 20% FBS-containing RPMI-1640 medium under 5% CO₂ at 37°C. Granta-519 cells at 1×10⁷ cells/0.1 mL were transplanted into 6 to 8-week-old CB17/SCID mice by intravenous injection. The day of the transplantation was defined as day 0. On day 1 after the transplantation, the mice were divided into groups depending on the body weight of each individual. The grouping was conducted using Matched distribution method (StudyDirector^{™} software, version 3.1.399.19).

Administration of the test substances was started on day 2 after the transplantation of the cancer cells. The doses of NB007-01, CAP-10, and a human IgG1 as an isotype control were 3 mg/kg, and the dose of defucosylated NB007-01 was 0.03 mg/kg. Each test substance was administered at a volume of 10 µL/g, and an administration frequency was twice a week.

After the transplantation of the cancer cells, the morbidity and mortality of the individuals were checked every day. Abnormalities of motor ability, food and water consumption, weight gain or loss, eye and fur conditions, and the like due to the tumor growth or influence of the treatment were checked. The body weight was measured twice a week. When death or clinical signs were confirmed, the details were recorded for each individual. The body weight measurement was conducted using Study Director^{™} software (version 3.1.399.19). The effect of the anti-tumor activity was evaluated based on the survival rates of individuals expressed in Kaplan-Meier survival curve.

The livers and lymph nodes of some individuals in each group were sampled on day 24 after the cancer cell transplantation. The sampled organs were immobilized with a 10% formalin buffer to prepare paraffin blocks. The prepared blocks were cut into 4 µm slices and treated at 60°C for 30 minutes. An infiltration ratio of Granta-519 cells was measured using an immunostaining method. An anti-human CD45 antibody (Cell Signaling Technology, Cat# 13917) as a hematopoietic cell surface marker was used to identify Granta-519 cells. The slices were activated with EDTA, pH 9.0, at 100°C for 20 minutes. A dilution ratio of the antibody used was 1:800. All of the completely immnostained slides were scanned at a magnification of 40 times using NanoZoomer-HT 2.0/Pannoramic SCAN Image system, and the scanned images were stored. For immunostaining scoring, the number of CD45-positive cells were counted using HALO^{™} platform, and evaluated as the number of the cells per 1 mm².

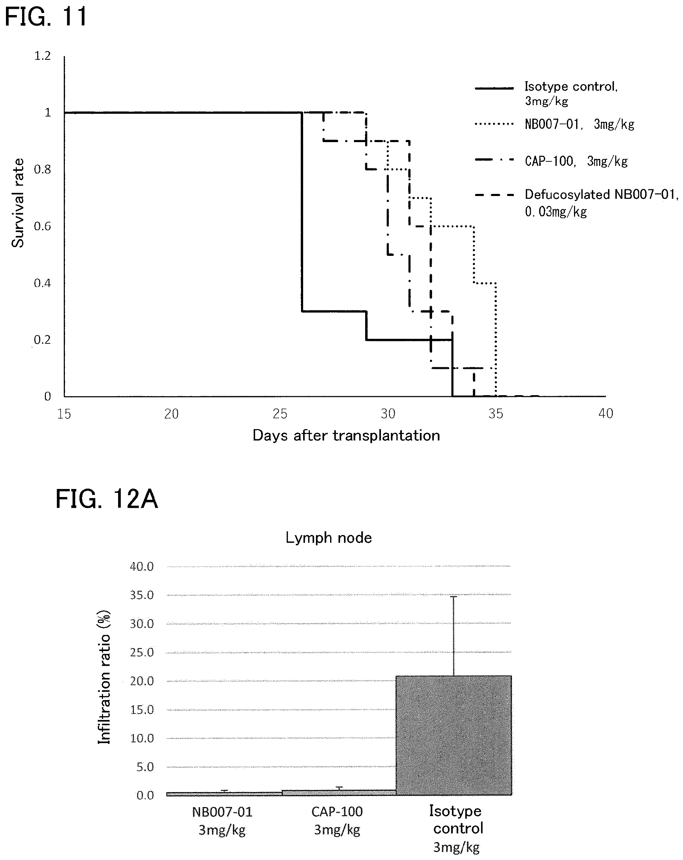

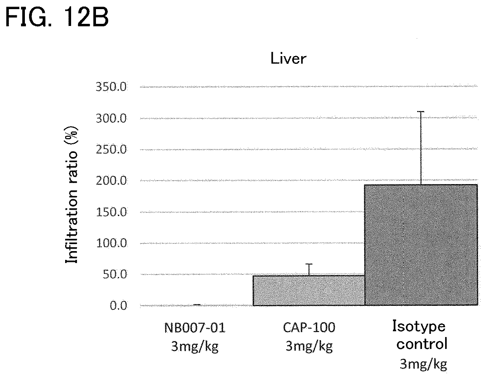

Fig. 11 presents transitions of the survival rates expressed in Kaplan-Meier survival curve. The number of the CD45-positive cells in the lymph nodes is presented in Fig. 12A, and the number of the CD45-positive cells in the liver is presented in Fig. 12B. Fig. 11 shows that the survival period of NB007-01 was extended compared to the isotype control group and further extended compared to CAP-100. As presented in Fig. 12A and Fig. 12B, NB007-01 strongly suppressed the infiltration of cancer cells into various organs and showed an effect stronger than that of CAP-100 in the liver. This suggested that NB007-01 exerted an anti-tumor effect by suppressing the infiltration of lymphoma into various organs. Furthermore, it was observed that the defucosylated NB007-01 had a survival extending effect equivalent to that of CAP-100, in one hundredth the dose of CAP-100. This indicated that defucosylated NB007-01 having an enhanced cytotoxicity activity could be expected to exert an anti-tumor effect at a low dose.

## Claims

1. An antibody that specifically binds to an extracellular domain of human CCR7, comprising:
a heavy chain CDR1 including an amino acid sequence represented by SEQ ID NO: 25;
a heavy chain CDR2 including an amino acid sequence represented by SEQ ID NO: 27;
a heavy chain CDR3 including an amino acid sequence represented by SEQ ID NO: 29;
a light chain CDR1 including an amino acid sequence represented by SEQ ID NO: 35;
a light chain CDR2 including an amino acid sequence represented by SEQ ID NO: 37; and
a light chain CDR3 including an amino acid sequence represented by SEQ ID NO: 39.

2. The antibody according to claim 1, comprising:
a heavy chain variable region including an amino acid sequence represented by SEQ ID NO: 21; and
a light chain variable region including an amino acid sequence represented by SEQ ID NO: 31.

3. The antibody according to claim 1, wherein
the antibody comprises an Fc region,
the Fc region comprises an N-glycoside-bound sugar chain,
the antibody does not comprise fucose bound to position 6 of N-acetylglucosamine on a reducing end of the N-glycoside-bound sugar chain, and
the antibody comprises an antibody-dependent cell-mediated cytotoxicity effect.

4. The antibody according to claim 2, wherein
the antibody comprises an Fc region,
the Fc region comprises an N-glycoside-bound sugar chain,
the antibody does not comprise fucose bound to position 6 of N-acetylglucosamine on a reducing end of the N-glycoside-bound sugar chain, and
the antibody comprises an antibody-dependent cell-mediated cytotoxicity effect.

5. An antibody that specifically binds to an extracellular domain of human CCR7, comprising:
a heavy chain variable region including an amino acid sequence represented by SEQ ID NO: 21 with 1 to 10 amino acids substituted, added, or deleted, or including an amino acid sequence having at least 90% identity with the amino acid sequence represented by SEQ ID NO: 21; and
a light chain variable region including an amino acid sequence represented by SEQ ID NO: 31 with 1 to 10 amino acids substituted, added, or deleted, or including an amino acid sequence having at least 90% identity with the amino acid sequence represented by SEQ ID NO: 31; wherein
the antibody comprises an Fc region,
the Fc region comprises an N-glycoside-bound sugar chain,
the antibody does not comprise fucose bound to position 6 of N-acetylglucosamine on a reducing end of the N-glycoside-bound sugar chain, and
the antibody comprises an antibody-dependent cell-mediated cytotoxicity effect.

6. An antibody that specifically binds to an extracellular domain of human CCR7, comprising:
a heavy chain CDR1 including an amino acid sequence represented by SEQ ID NO: 45;
a heavy chain CDR2 including an amino acid sequence represented by SEQ ID NO: 47;
a heavy chain CDR3 including an amino acid sequence represented by SEQ ID NO: 49;
a light chain CDR1 including an amino acid sequence represented by SEQ ID NO: 55;
a light chain CDR2 including an amino acid sequence represented by SEQ ID NO: 57, and
a light chain CDR3 including an amino acid sequence represented by SEQ ID NO: 59.

7. The antibody according to claim 6, comprising:
a heavy chain variable region including an amino acid sequence represented by SEQ ID NO: 41; and
a light chain variable region including an amino acid sequence represented by SEQ ID NO: 51.

8. The antibody according to claim 6, wherein
the antibody comprises an Fc region,
the Fc region comprises an N-glycoside-bound sugar chain,
the antibody does not comprise fucose bound to position 6 of N-acetylglucosamine on a reducing end of the N-glycoside-bound sugar chain, and
the antibody comprises an antibody-dependent cell-mediated cytotoxicity effect.

9. The antibody according to claim 7, wherein
the antibody comprises an Fc region,
the Fc region comprises an N-glycoside-bound sugar chain,
the antibody does not comprise fucose bound to position 6 of N-acetylglucosamine on a reducing end of the N-glycoside-bound sugar chain, and
the antibody comprises an antibody-dependent cell-mediated cytotoxicity effect.

10. An antibody that specifically binds to an extracellular domain of human CCR7, comprising:
a heavy chain variable region including an amino acid sequence represented by SEQ ID NO: 41 with 1 to 10 amino acids substituted, added, or deleted, or including an amino acid sequence having at least 90% identity with the amino acid sequence represented by SEQ ID NO: 41; and
a light chain variable region including an amino acid sequence represented by SEQ ID NO: 51 with 1 to 10 amino acids substituted, added, or deleted, or including an amino acid sequence having at least 90% identity with the amino acid sequence represented by SEQ ID NO: 51; wherein
the antibody comprises an Fc region,
the Fc region comprises an N-glycoside-bound sugar chain,
the antibody does not comprise fucose bound to position 6 of N-acetylglucosamine on a reducing end of the N-glycoside-bound sugar chain, and
the antibody comprises an antibody-dependent cell-mediated cytotoxicity effect.

11. A nucleic acid encoding the antibody according to any one of claims 1 to 10.

12. The nucleic acid according to claim 11, comprising a first nucleic acid encoding an amino acid sequence represented by SEQ ID NO: 21 and a second nucleic acid encoding an amino acid sequence represented by SEQ ID NO: 31.

13. The nucleic acid according to claim 11, comprising a first nucleic acid encoding an amino acid sequence represented by SEQ ID NO: 41 and a second nucleic acid encoding an amino acid sequence represented by SEQ ID NO: 51.

14. A cell comprising the nucleic acid according to claim 11.

15. A cell comprising the nucleic acid according to claim 12.

16. A cell comprising the nucleic acid according to claim 13.

17. A medicament comprising the antibody according to any one of claims 1 to 10 as an active ingredient.

18. The medicament according to claim 17, which is used for treating a cancer.

19. The medicament according to claim 18, wherein the cancer is a hematological cancer.

20. The medicament according to claim 19, wherein the hematological cancer is acute myeloid leukemia, acute lymphocytic leukemia, chronic myeloid leukemia, chronic lymphocytic leukemia, or non-Hodgkin's lymphoma.

21. The medicament according to claim 20, wherein the non-Hodgkin's lymphoma is B cell lymphoblastic leukemia, T cell lymphoblastic leukemia, chronic lymphocytic leukemia, follicular lymphoma, MALT lymphoma, lymphoplasmacytic lymphoma, mantle cell lymphoma, diffuse large B cell lymphoma, Burkitt's lymphoma, mature T/NK cell-derived peripheral T cell lymphoma, adult T cell leukemia, extranodal NK/T cell lymphoma, or cutaneous lymphoma.

22. The medicament according to claim 18, wherein the cancer is a solid carcinoma.

23. The medicament according to claim 22, wherein the solid carcinoma is breast cancer, malignant breast tumor, gastric cancer, melanoma, non-small-cell lung cancer, pulmonary adenocarcinoma, cervical cancer, hepatoma, urothelial cancer, renal cell cancer, or squamous cell cancer.

24. The medicament according to claim 23, wherein the squamous cell cancer is oral squamous cell carcinoma, esophageal squamous cell carcinoma, or pharyngeal squamous cell carcinoma.
